# EUROPEAN PATENT SPECIFICATION

(11) **EP 4 313 060 B1**
(45) Date of publication and mention of the grant of the patent: **05.03.2025**
(21) Application number: 22716719.4
(22) Date of filing: 25.03.2022
(51) Int. Cl.: A61K 31/538, A61K 38/14, A61P 31/04

(54) **RIFAMYCIN ANALOGS IN COMBINATION WITH VANCOMYCIN AND USES THEREOF**
RIFAMYCIN-ANALOGE IN KOMBINATION MIT VANCOMYCIN UND VERWENDUNGEN DAVON
ANALOGUES DE LA RIFAMYCINE EN COMBINAISON AVEC LA VANCOMYCINE ET UTILISATIONS DE CEUX-CI

(30) Priority: 26.03.2021 US 202163166597 P
(43) Date of publication of application: 07.02.2024
(73) Proprietor: Regeneron Pharmaceuticals, Inc., Tarrytown, NY 10591 (US)
(72) Inventor: NITTOLI, Thomas, Tarrytown, NY 10591 (US); ZUMSTEG, Anna, Kirkwood, MO 63122 (US)
(74) Representative: Carpmaels & Ransford LLP
(86) International application number: PCT/US2022/021922
(87) International publication number: WO 2022/204499

(56) References cited:
- WO-A1-2019/217591
- WO-A1-2020/132483

## Description

### CROSS-REFERENCE TO RELATED APPLICATIONS

This application claims priority to U.S. Provisional Patent Application Serial No. 63/166,597 filed March 26, 2021.

### FIELD OF DISCLOSURE

The present disclosure relates to rifamycin analog compounds used in combination with vancomycin for inhibiting bacterial growth and treating bacterial infections, as well as and pharmaceutical compositions and dosage forms comprising rifamycin analogs and vancomycin, and methods of use thereof.

### BACKGROUND OF THE DISCLOSURE

*Staphylococcus aureus* (*S. aureus*) is a Gram-positive, round-shaped bacterium that is a member of the Firmicutes, and it is a usual member of the microbiota of the body, frequently found in the upper respiratory tract and on the skin. It is often positive for catalase and nitrate reduction and is a facultative anaerobe that can grow without the need for oxygen. Although S. *aureus* usually acts as a commensal of the human microbiota, it can also become an opportunistic pathogen, being a common cause of skin infections including abscesses, respiratory infections such as sinusitis, and food poisoning. Pathogenic strains often promote infections by producing virulence factors such as potent protein toxins, and the expression of a cell-surface protein that binds and inactivates antibodies.

An estimated 20% to 30% of the human population are long-term carriers of S. *aureus,* which can be found as part of the normal skin flora, in the nostrils, and as a normal inhabitant of the lower reproductive tract of women. *S. aureus* can cause a range of illnesses, from minor skin infections, such as pimples, impetigo, boils, cellulitis, folliculitis, carbuncles, scalded skin syndrome, and abscesses, to life-threatening diseases such as pneumonia, meningitis, osteomyelitis, endocarditis, toxic shock syndrome, bacteremia, and sepsis. It is still one of the five most common causes of hospital-acquired infections and is often the cause of wound infections following surgery. Each year, around 500,000 patients in hospitals of the United States contract a staphylococcal infection, chiefly by *S. aureus.* Up to 50,000 deaths each year in the USA are linked with *S. aureus* infections. Schlecht LM et al, 2015, Microbiology, 161, 1, 168-181. Despite much research and development, no vaccine for *S. aureus* has been approved at present.

Initially, the treatment of choice for *S. aureus* infection was penicillin. Antibiotic **resistance** in *S. aureus* was uncommon when penicillin was first introduced in 1943. By 1950, 40% of hospital *S. aureus* isolates were penicillin-resistant; by 1960, this had risen to 80%. Chambers HF, 2001, Emerging Infectious Diseases, 7, 2, 178-82. Today, *S. aureus* has become resistant to many commonly used antibiotics.

The emergence of antibiotic-resistant strains of *S. aureus* such as methicillin-resistant *S. aureus* (MRSA) is a worldwide problem in clinical medicine. MRSA strains are most often found associated with institutions such as hospitals, but are becoming increasingly prevalent in community-acquired infections. MRSA is one of a number of greatly feared strains of *S. aureus* which have become resistant to most β-lactam antibiotics. MRSA infections in both the hospital and community setting are commonly treated with non-β-lactam antibiotics, such as clindamycin (a lincosamine) and co-trimoxazole (also commonly known as trimethoprim/sulfamethoxazole). Resistance to these antibiotics has also led to the use of new, broad-spectrum anti-Gram-positive antibiotics, such as linezolid, because of its availability as an oral drug. First-line treatment for serious invasive infections due to MRSA is currently glycopeptide antibiotics (vancomycin and teicoplanin). A number of problems with these antibiotics occur, such as the need for intravenous administration (no oral preparation is available), toxicity, and the need to monitor drug levels regularly by blood tests. Also, glycopeptide antibiotics do not penetrate very well into infected tissues (this is a particular concern with infections of the brain and meninges and in endocarditis). Thus, there exists a strong unmet need for novel antibiotic treatments for *S. aureus* in general, and in addressing intracellular *S. aureus* infections in particular.

Rifamycins, a subclass of the ansamycin antibiotic family, are a group of antibiotics that are synthesized either naturally by the bacterium *Amycolatopsis rifamycinica* or artificially. Rifamycins are particularly effective against mycobacteria, and are therefore used to treat tuberculosis, leprosy, and mycobacterium avium complex (MAC) infections. The rifamycin group includes the "classic" rifamycin drugs as well as the rifamycin analogs rifampicin (or rifampin), rifabutin, rifapentine, rifalazil and rifaximin. Rifamycin SV, sold under the trade name Aemcolo, is FDA-approved for treatment of travelers' diarrhea in some circumstances.

Rifamycin class antibiotics inhibit bacterial RNA polymerase (RNAP) and have potent activity against *S. aureus.* Monotherapy with this class of antibiotics, however, can lead to selection of a resistant population during treatment. Therefore, rifamycin antibiotics can be used in combination with first line antibiotics to improve outcomes, commonly in infections involving prostheses or foreign devices.

Vancomycin is a very important antibiotic for the treatment of Gram-positive infections, including *S. aureus* infections. It has been proposed that vancomycin works by binding to the D-ala-D-ala termini of Lipid II molecules and/or to nascent peptidoglycan, preventing maturation of the cell wall. Vancomycin is used to treat infections that are resistant to antibiotics, including antibiotic-resistance *S. aureus* infections. Hence, vancomycin has been called the "antibiotic of last resort" because it is often the last remaining option for the treatment of Gram-positive infections caused by strains that have become resistant to all other antibiotics. Resistance to vancomycin first appeared about fifteen years ago and the frequency of resistance is increasing. There have been numerous efforts to develop further treatments to combat the emergent vancomycin-resistant strains.

Thus, there exists a strong unmet need for developing effective antibiotics in order to combat the growing problem of antibiotic-resistant bacteria, including antibiotic-resistant S. *aureus* strains. There also exists a need of enhancing efficacy of vancomycin and other existing antibiotics. WO2020/132483 discloses rifamycin analog compounds of Formula (A), which can be used in combination with a second antibiotic, such as vancomycin, for inhibiting bacterial growth and treating bacterial infections. The presently claimed compounds of formula (I) and (II) are also disclosed.

The foregoing discussion is presented solely to provide a better understanding of the nature of the problems confronting the art and should not be construed in any way as an admission as to prior art nor should the citation of any reference herein be construed as an admission that such reference constitutes "prior art" to the instant application.

### SUMMARY OF THE DISCLOSURE

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy (or for diagnosis).

As discussed herein, there is a strong need to develop effective treatments for bacterial infections in general and *S. aureus* infections in particular. The present disclosure addresses these and other needs by providing combinations of rifamycin analog compounds and vancomycin, as well as pharmaceutical compositions and dosage forms comprising same, and methods of treatment based on such compounds and pharmaceutical compositions.

In one aspect, the present disclosure provides a method of preventing or inhibiting growth of a bacterium comprising administering an effective amount of:
a) a compound selected from 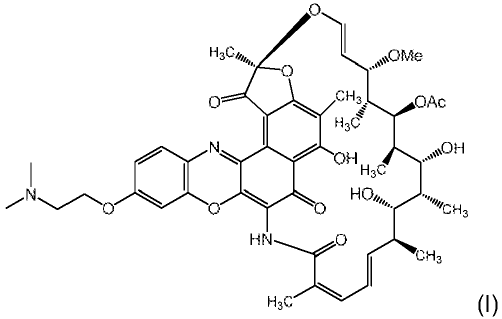 and 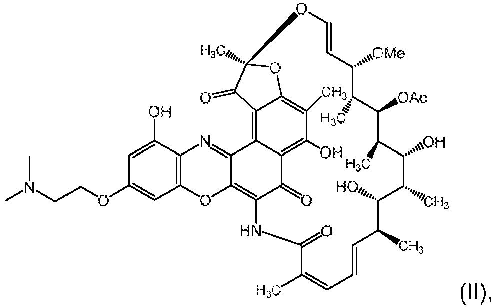 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the compound of a) is compound (I) or a pharmaceutically acceptable salt thereof. In one embodiment, the compound of a) is compound (II) or a pharmaceutically acceptable salt thereof.

In one embodiment, the bacterium is a Gram-positive bacterium. In one embodiment, the bacterium is a penicillin-resistant bacterium, or a methicillin-resistant bacterium.

In one embodiment, the bacterium is *Staphylococcus aureus.* In one embodiment, the bacterium is selected from methicillin-resistant *Staphylococcus aureus* (MRSA) and methicillin-susceptible *Staphylococcus aureus* (MSSA). In one embodiment, the bacterium is methicillin-resistant *Staphylococcus aureus* (MRSA).

In another aspect, the present disclosure provides a method of treating a bacterial infection in a subject in need of such treatment comprising administering to the subject an effective amount of:
a) a compound selected from 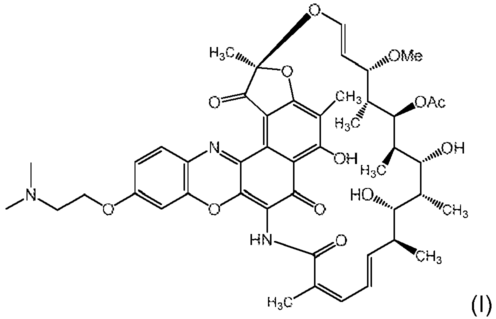 and 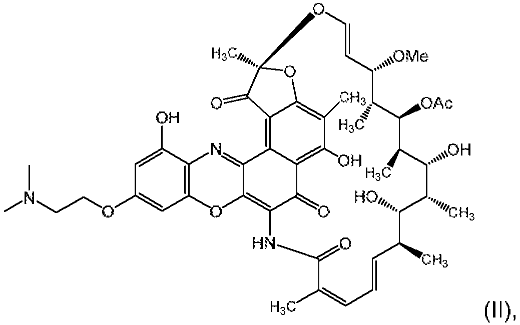 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the compound of a) is compound (I) or a pharmaceutically acceptable salt thereof. In one embodiment, the compound of a) is compound (II) or a pharmaceutically acceptable salt thereof.

In one embodiment, the bacterial infection is caused by a Gram-positive bacterium. In one embodiment, the bacterial infection is caused by a penicillin-resistant bacterium or a methicillin-resistant bacterium.

In one embodiment, wherein the bacterial infection is caused by *Staphylococcus aureus.* In one embodiment, the bacterial infection is caused by a bacterium selected from methicillin-resistant *Staphylococcus aureus* (MRSA) and methicillin-susceptible *Staphylococcus aureus* (MSSA). In one embodiment, the bacterial infection is caused by methicillin-resistant *Staphylococcus aureus* (MRSA).

In one embodiment, the bacterial infection is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

In another aspect, the present disclosure provides a method of preventing or treating a disease in a subject in need thereof comprising administering to the subject an effective amount of a) a compound selected from 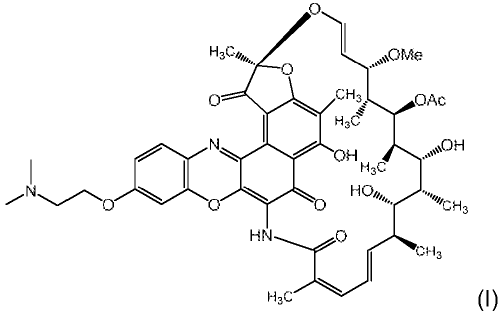 and 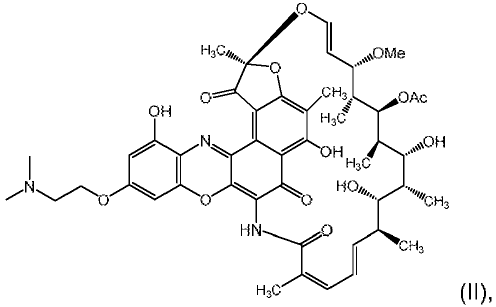 or a pharmaceutically acceptable salt thereof, and
b) vancomycin or an ester, amide, N-oxide, a pharmaceutically acceptable salt of an ester or amide, or a pharmaceutically acceptable salt thereof, wherein the disease is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

In another aspect, the present disclosure provides a method of enhancing efficacy of vancomycin or an ester, amide, N-oxide, a pharmaceutically acceptable salt of an ester or amide thereof comprising administering to a subject in need thereof an effective amount of:
a) a compound selected from 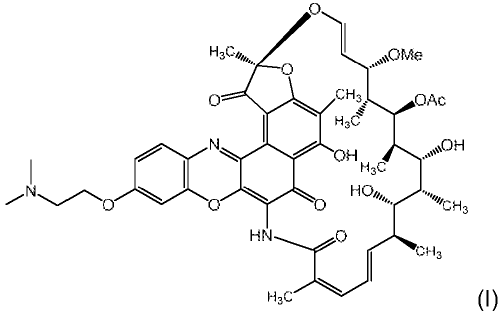 and 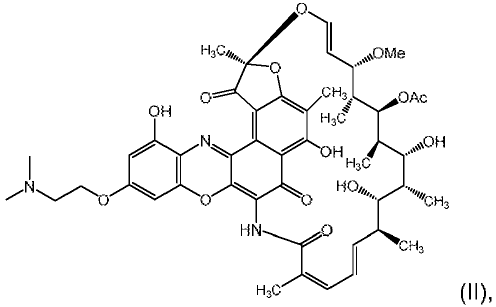 or a pharmaceutically acceptable salt thereof, in combination with
b) vancomycin or an ester, amide, N-oxide, a pharmaceutically acceptable salt of an ester or amide, or a pharmaceutically acceptable salt thereof.

In yet another aspect, the present disclosure provides a method of enhancing efficacy of a compound selected from 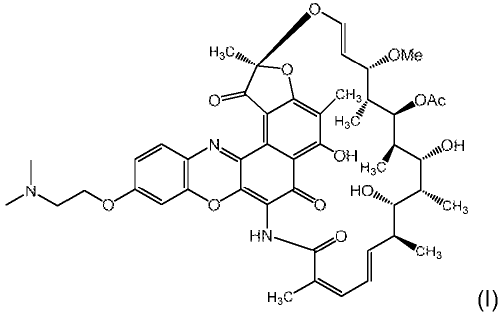 and 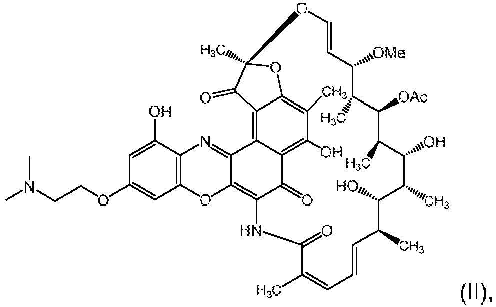 or a pharmaceutically acceptable salt thereof, the method comprising administering a subject in need thereof an effective amount of the compound (I) or (II) in combination with vancomycin or an ester, amide, N-oxide, a pharmaceutically acceptable salt of an ester or amide, or a pharmaceutically acceptable salt thereof.

In one embodiment, the compound (I) or (II), or a pharmaceutically acceptable salt thereof, is administered orally, subcutaneously, or intravenously. In one embodiment, the vancomycin or an ester, amide, N-oxide, a pharmaceutically acceptable salt of an ester or amide, or a pharmaceutically acceptable salt thereof, is administered orally, subcutaneously, or intravenously.

In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered in a single dosage form. In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered in separate dosage forms.

In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on the same day. In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered simultaneously.

In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are not administered on the same day. In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on consecutive days.

In one embodiment, the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on separate, non-consecutive days.

In one embodiment, the vancomycin or an ester, amide, N-oxide, a pharmaceutically acceptable salt of an ester or amide, or a pharmaceutically acceptable salt thereof, is administered twice daily. In one embodiment, the compound (I) or (II), or a pharmaceutically acceptable salt thereof, is administered once daily.

In one embodiment, the subject is human.

In one aspect, the present disclosure provides a pharmaceutical composition comprising a) a compound selected from 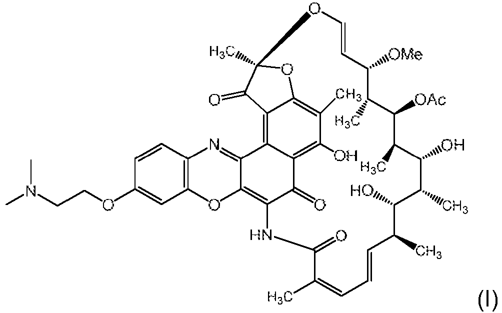 and 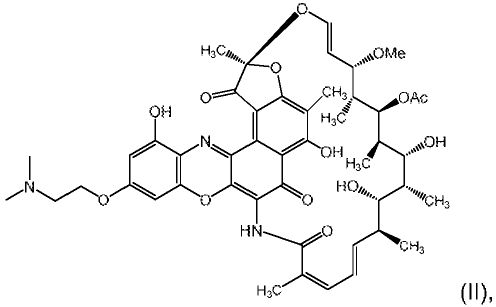 or a pharmaceutically acceptable salt thereof,
b) vancomycin or an ester, amide, N-oxide, a pharmaceutically acceptable salt of an ester or amide, or a pharmaceutically acceptable salt thereof, and
c) a pharmaceutically acceptable carrier.

In one embodiment, the pharmaceutical composition comprises from about 0.01 mg to about 5000 mg of vancomycin or an ester, amide, N-oxide, a pharmaceutically acceptable salt of an ester or amide thereof.

In one embodiment, the pharmaceutical composition comprises from about 0.001 mg to about 5000 mg of the compound (I) or (II). In one embodiment, the pharmaceutical composition comprises from about 0.01 mg to about 500 mg of the compound (I) or (II).

A pharmaceutical dosage form comprising a) a compound selected from 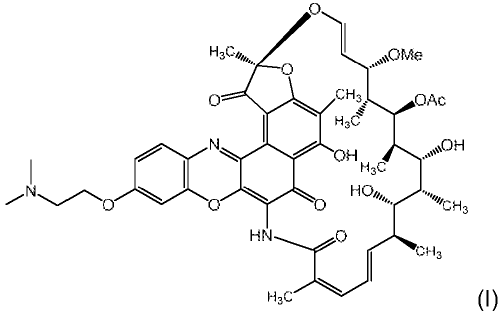 and 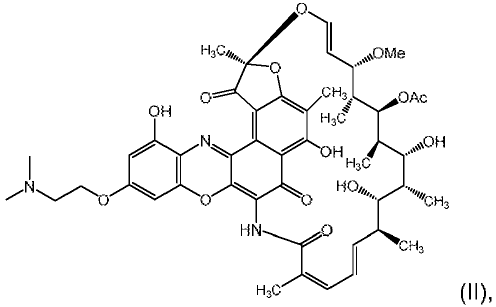 or a pharmaceutically acceptable salt thereof,
b) vancomycin or an ester, amide, N-oxide, a pharmaceutically acceptable salt of an ester or amide, or a pharmaceutically acceptable salt thereof, and
c) a pharmaceutically acceptable vehicle.

In one embodiment, the pharmaceutical dosage form comprises the dosage form is an oral dosage form or an injectable dosage form.

In one embodiment, the pharmaceutical dosage form comprises from about 0.01 mg to about 5000 mg of vancomycin or an ester, amide, N-oxide, a pharmaceutically acceptable salt of an ester or amide thereof. In one embodiment, the pharmaceutical dosage form comprises from about 0.001 mg to about 5000 mg of the compound (I) or (II). In one embodiment, the pharmaceutical dosage form comprises from about 0.01 mg to about 500 mg of the compound (I) or (II).

In one embodiment, the pharmaceutical dosage form is formulated for a single administration. In one embodiment, the pharmaceutical dosage form is formulated for multiple administrations.

In yet another aspect, the present disclosure provides a kit comprising a) a first container comprising a compound selected from 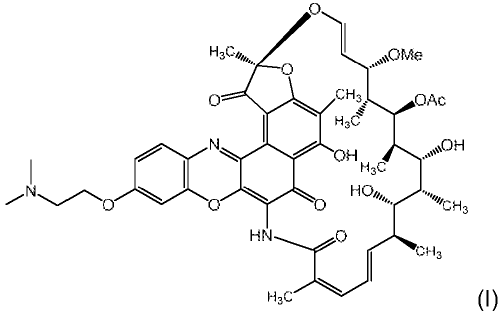 and 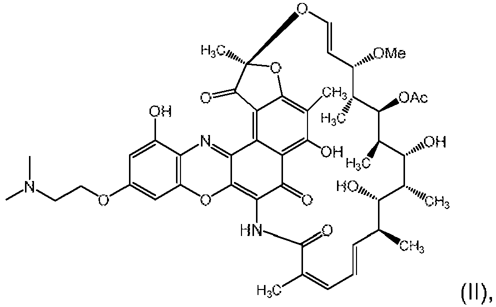 or a pharmaceutically acceptable salt thereof,
b) a second container comprising vancomycin or an ester, amide, N-oxide, a pharmaceutically acceptable salt of an ester or amide, or a pharmaceutically acceptable salt thereof, and
c) instructions for administration of the compound (I) or (II) and the vancomycin or an ester, amide, N-oxide, a pharmaceutically acceptable salt of an ester or amide thereof.

In one embodiment, the first container comprises compound (I) or a pharmaceutically acceptable salt thereof. In one embodiment, the first container comprises compound (II) or a pharmaceutically acceptable salt thereof.

These and other aspects of the present disclosure will become apparent to those skilled in the art after a reading of the following detailed description of the disclosure, including the appended claims.

### BRIEF DESCRIPTION OF THE DRAWINGS

**Figure 1** (reference) is a bar graph of the median *S. aureus* NRS384 kidney burden in mice treated with rifampicin in combination with vancomycin.
**Figure 2** is a bar graph of the median *S. aureus* NRS384 kidney burden in mice treated with rifamycin analogs (I) and (II) according to the disclosure in combination with vancomycin.
Figure 3 is a bar graph of the median *S. aureus* NRS384 kidney burden in mice treated with rifampicin (reference) or rifamycin analog (II) according to the disclosure, with or without combination with vancomycin.
Figure 4 is a bar graph of the median *S. aureus* N315 kidney burden in mice treated with rifampicin (reference) or rifamycin analogs (I) and (II) according to the disclosure in combination with vancomycin.

### DETAILED DESCRIPTION

Detailed embodiments of the present disclosure are disclosed herein.

In addition, each of the examples given in connection with the various embodiments of the disclosure is intended to be illustrative, and not restrictive. Therefore, specific structural and functional details disclosed herein are not to be interpreted as limiting, but merely as a representative basis for teaching one skilled in the art to variously employ the present disclosure.

### Definitions

Unless defined otherwise, all technical and scientific terms used herein have the same meaning as commonly understood by one of ordinary skill in the art to which this disclosure belongs.

As used in this specification and the appended claims, the singular forms "a", "an", and "the" include plural references unless the context clearly dictates otherwise. Thus, for example, a reference to "a method" includes one or more methods, and/or steps of the type described herein and/or which will become apparent to those persons skilled in the art upon reading this disclosure.

The terms "treat" or "treatment" of a state, disorder or condition include: (1) preventing, delaying, or reducing the incidence and/or likelihood of the appearance of at least one clinical or sub-clinical symptom of the state, disorder or condition developing in a subject that may be afflicted with or predisposed to the state, disorder or condition but does not yet experience or display clinical or subclinical symptoms of the state, disorder or condition; or (2) inhibiting the state, disorder or condition, i.e., arresting, reducing or delaying the development of the disease or a relapse thereof or at least one clinical or sub-clinical symptom thereof; or (3) relieving the disease, i.e., causing regression of the state, disorder or condition or at least one of its clinical or sub-clinical symptoms. The benefit to a subject to be treated is either statistically significant or at least perceptible to the patient or to the physician.

A "subject" or "patient" or "individual" or "animal", as used herein, refers to humans, veterinary animals (e.g., cats, dogs, cows, horses, sheep, pigs, etc.) and experimental animal models of diseases (e.g., mice, rats). In one embodiment, the subject is a human.

As used herein the term "effective" applied to dose or amount refers to that quantity of a compound or pharmaceutical composition that is sufficient to result in a desired activity upon administration to a subject in need thereof. Note that when a combination of active ingredients is administered, the effective amount of the combination may or may not include amounts of each ingredient that would have been effective if administered individually. The exact amount required will vary from subject to subject, depending on the species, age, and general condition of the subject, the severity of the condition being treated, the particular drug or drugs employed, the mode of administration, and the like.

The phrase "pharmaceutically acceptable", as used in connection with compositions of the disclosure, refers to molecular entities and other ingredients of such compositions that are physiologically tolerable and do not typically produce untoward reactions when administered to a mammal (e.g., a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The phrase "therapeutically effective amount," as used herein, refers to an amount that produces the desired effect for which it is administered. The exact amount will depend on the purpose of the treatment, and will be ascertainable by one skilled in the art using known techniques (see, for example, Lloyd (1999) The Art, Science and Technology of Pharmaceutical Compounding).

Ranges can be expressed herein as from "about" or "approximately" one particular value and/or to "about" or "approximately" another particular value. When such a range is expressed, another embodiment includes from the one particular value and/or to the other particular value.

By "comprising" or "containing" or "including" is meant that at least the named compound, element, particle, or method step is present in the composition or article or method, but does not exclude the presence of other compounds, materials, particles, or method steps, even if the other such compounds, material, particles, or method steps have the same function as what is named.

Compounds of the present disclosure include those described generally herein, and are further illustrated by the classes, subclasses, and species disclosed herein. As used herein, the following definitions shall apply unless otherwise indicated. For purposes of this disclosure, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

The chemistry of protecting groups can be found, for example, in Wuts and Greene, Greene's Protective Groups in Organic Synthesis, 4th Ed., John Wiley & Sons: New York, 2006.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the disclosure.

Unless otherwise stated, all tautomeric forms of the compounds of the disclosure are within the scope of the disclosure.

Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹¹C- or ¹³C- or ¹⁴C -enriched carbon, or the replacement of an oxygen by a ¹⁷O- or ¹⁸O-enriched oxygen, or the replacement of a nitrogen by a ¹⁵N-enriched nitrogen are within the scope of this disclosure.

It is also to be understood that the mention of one or more method steps does not preclude the presence of additional method steps or intervening method steps between those steps expressly identified. Similarly, it is also to be understood that the mention of one or more components in a device or system does not preclude the presence of additional components or intervening components between those components expressly identified.

Unless otherwise stated, all crystalline forms of the compounds of the disclosure and salts thereof are also within the scope of the disclosure. The compounds of the disclosure may be isolated in various amorphous and crystalline polymorphic forms, including without limitation amorphous and crystalline polymorphic forms which are anhydrous, hydrated, non-solvated, or solvated. Example hydrates include hemihydrates, monohydrates, dihydrates, and the like. In some embodiments, the compounds of the disclosure are anhydrous and non-solvated. By "anhydrous" is meant that the crystalline form of the compound contains essentially no bound water in the crystal lattice structure, i.e., the compound does not form a crystalline hydrate.

As used herein, "crystalline form" is meant to refer to a certain lattice configuration of a crystalline substance. Different crystalline forms (polymorphic forms) of the same substance typically have different crystalline lattices (e.g., unit cells) which are attributed to different physical properties that are characteristic of each of the crystalline forms. In some instances, different lattice configurations have different water or solvent content. The different crystalline lattices can be identified by solid state characterization methods such as by X-ray powder diffraction (PXRD). Other characterization methods such as differential scanning calorimetry (DSC), thermogravimetric analysis (TGA), dynamic vapor sorption (DVS), solid state NMR, and the like further help identify the crystalline form as well as help determine stability and solvent/water content.

Crystalline forms of a substance include both solvated (e.g., hydrated) and non-solvated (e.g., anhydrous) forms. A hydrated form is a crystalline form that includes water in the crystalline lattice. Hydrated forms can be stoichiometric hydrates, where the water is present in the lattice in a certain water/molecule ratio such as for hemihydrates, monohydrates, dihydrates, etc. Hydrated forms can also be non-stoichiometric, where the water content is variable and dependent on external conditions such as humidity.

In some embodiments, the compounds of the disclosure are substantially isolated. By "substantially isolated" is meant that a particular compound is at least partially isolated from impurities. For example, in some embodiments a compound of the disclosure comprises less than about 50%, less than about 40%, less than about 30%, less than about 20%, less than about 15%, less than about 10%, less than about 5%, less than about 2.5%, less than about 1%, or less than about 0.5% of impurities. Impurities generally include anything that is not the substantially isolated compound including, for example, other crystalline forms and other substances.

As used herein, the term "antibiotic" (abx or Abx) includes any molecule that specifically inhibits the growth of or kills micro-organisms, such as bacteria, but is non-lethal to the host at the concentration and dosing interval administered. In a specific aspect, an antibiotic is non-toxic to the host at the administered concentration and dosing intervals. Antibiotics effective against bacteria can be broadly classified as either bactericidal (i.e., directly kills) or bacteriostatic (i.e., prevents division). Anti-bactericidal antibiotics can be further subclassified as narrow-spectrum or broad-spectrum. A broad-spectrum antibiotic is one effective against a broad range of bacteria including both Gram-positive and Gram-negative bacteria, in contrast to a narrow-spectrum antibiotic, which is effective against a smaller range or specific families of bacteria. Examples of antibiotics include: aminoglycosides, e.g., amikacin, gentamicin, kanamycin, neomycin, netilmicin, streptomycin, tobramycin, paromycin, ansamycins, e.g., geldanamycin, herbimycin, carbacephems, e.g., loracarbef, carbapenems, e.g., ertapenum, doripenem, imipenem/cilastatin, meropenem, cephalosporins (first generation), e.g., cefadroxil, cefazolin, cefalotin, cefalexin, cephalosporins (second generation), e.g., ceflaclor, cefamandole, cefoxitin, cefprozil, cefuroxime, cephalosporins (third generation), e.g., cefixime, cefdinir, cefditoren, cefoperazone, cefotaxime, cefpodoxime, ceftazidime, ceftibuten, ceftizoxime, ceftriaxone, cephalosporins (fourth generation), e.g., cefepime, cephalosporins (fifth generation), e.g., ceftobiprole, glycopeptides, e.g., teicoplanin, vancomycin, macrolides, e.g., axithromycin, clarithromycin, dirithromycine, erythromycin, roxithromycin, troleandomycin, telithromycin, spectinomycin, monobactams, e.g., axtreonam, penicilins, e.g., amoxicillin, ampicillin, axlocillin, carbenicillin, cloxacillin, dicloxacillin, flucloxacillin, mezlocillin, meticillin, nafcilin, oxacillin, penicillin, peperacillin, ticarcillin, antibiotic polypeptides, e.g., bacitracin, colistin, polymyxin B, quinolones, e.g., ciprofloxacin, enoxacin, gatifloxacin, levofloxacin, lemefloxacin, moxifloxacin, norfloxacin, orfloxacin, trovafloxacin, sulfonamides, e.g., mafenide, prontosil, sulfacetamide, sulfamethizole, sulfanilamide, sulfasalazine, sulfisoxazole, trimethoprim, trimethoprim-sulfamethoxazole (TMP-SMX), tetracyclines, e.g., demeclocycline, doxycycline, minocycline, oxytetracycline, tetracycline and others such as arspenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin/rifampicin or timidazole.

The term "methicillin-resistant *Staphylococcus aureus"* (MRSA), alternatively known as multidrug resistant *Staphylococcus aureus* or oxacillin-resistant *Staphylococcus aureus* (ORSA), refers to any strain of *Staphylococcus aureus* that is resistant to beta-lactam antibiotics, which include the penicillins (e.g., methicillin, dicloxacillin, nafcillin, oxacillin, etc.) and the cephalosporins. "Methicillinsensitive *Staphylococcus aureus"* (MSSA) refers to any strain of Staphylococcus aureus that is sensitive to betalactam antibiotics.

The term "vancomycin derivative" as used herein refers to a compound having a structure derived from vancomycin, which exhibits the same or substantially similar biological activity and physicochemical properties as vancomycin. Examples include, but are not limited to, salts, esters, amides, salts of esters or amides, and N-oxides of vancomycin. In the context of the disclosures herein, reference to the biological activity of vancomycin is reference to its inhibitory or biocidal activity on Gram-positive bacterial growth.

### Compounds of the Disclosure

In accordance with the foregoing objective and others, the present disclosure provides rifamycin analog compounds in combination with vancomycin, and pharmaceutically acceptable salts thereof as well as, pharmaceutical compositions and dosage forms comprising same and methods for inhibiting bacterial growth and/or treating a bacterial infection in a subject in need of such treatment.

In one aspect, the present disclosure provides rifamycin analogs (I) and (II) according to the following structures: 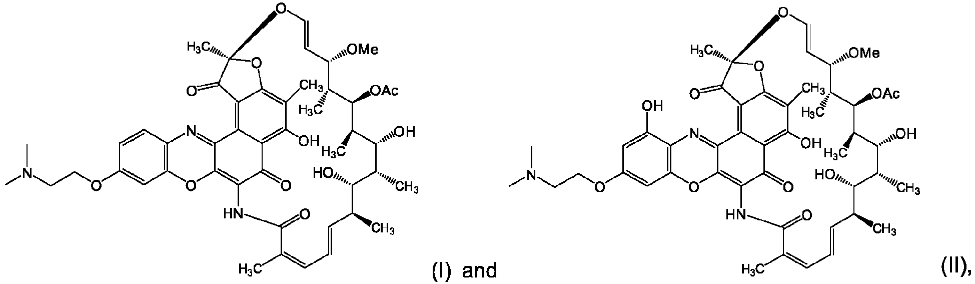 or pharmaceutically acceptable salts thereof, in combination with vancomycin, or a pharmaceutically acceptable salt thereof.

Vancomycin is an antibiotic medication used to treat a number of bacterial infections, e.g., as a treatment for complicated skin infections, bloodstream infections, endocarditis, bone and joint infections, and meningitis caused by S. *aureus,* e.g., MRSA. Vancomycin has the following chemical structure:

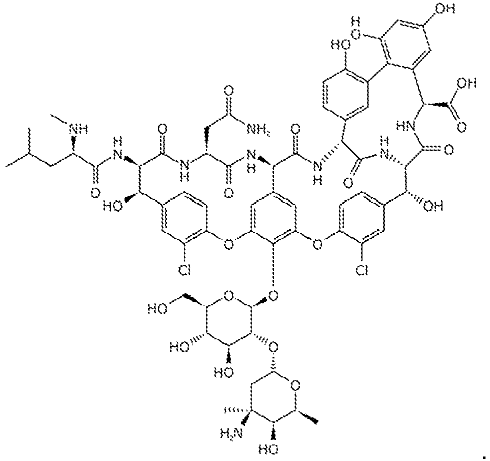

In one embodiment, a rifamycin analog is used in combination with vancomycin. In another embodiment, a rifamycin analog is used in combination with a derivative of vancomycin as defined in the claims. In one embodiment, the vancomycin derivative as used herein has a structure derived from vancomycin, which exhibits the same or substantially similar biological activity and physicochemical properties as vancomycin. Examples include salts, esters, amides, salts of esters or amides, and N-oxides of vancomycin. In the context of the disclosures herein, reference to the biological activity of vancomycin is reference to its inhibitory or biocidal activity on Gram-positive bacterial growth.

In one embodiment, the rifamycin analog used in combination with vancomycin or a derivative thereof as defined in the claims, is rifamycin analog (I), or pharmaceutically acceptable salts thereof.

In one embodiment, the rifamycin analog used in combination with vancomycin is rifamycin analog (II), or pharmaceutically acceptable salts thereof.

In one aspect, the rifamycin analog is used in combination with vancomycin or a derivative thereof as defined in the claims. and further in combination with a third active agent, e.g., a third antibiotic agent.

In one embodiment, the third antibiotic is effective against Staphylococcus aureus.

In one embodiment, the third antibiotic is selected from an aminoglycoside, a beta-lactam, a macrolide, a cyclic peptide, a tetracycline, a fluoroquinoline, a fluoroquinolone, and an oxazolidinone.

The present disclosure also includes salts of the compounds described herein. As used herein, "salts" refers to derivatives of the disclosed compounds wherein the parent compound is modified by converting an existing acid or base moiety to its salt form. Examples of salts include, but are not limited to, mineral acid (such as HC1, HBr, H₂SO₄) or organic acid (such as acetic acid, benzoic acid, trifluoroacetic acid salts of basic residues such as amines; alkali (such as Li, Na, K, Mg, Ca) or organic (such as trialkylammonium) salts of acidic residues such as carboxylic acids; and the like. The salts of the present application can be synthesized from the parent compound which contains a basic or acidic moiety conventional chemical methods. Generally, such salts can be prepared by reacting the free acid or base forms of these compounds with a stoichiometric amount of the appropriate base or acid in water or in an organic solvent, or in a mixture of the two. In some embodiments, non-aqueous media like ether, ethyl acetate, ethanol, isopropanol, or acetonitrile (ACN) may be used.

The present application also includes pharmaceutically acceptable salts of the compounds described herein. The "pharmaceutically acceptable salts" include a subset of the "salts" described above which are conventional non-toxic salts of the parent compound formed, for example, from non-toxic inorganic or organic acids. Lists of suitable salts are found in Remington's Pharmaceutical Sciences, 17th ed., Mack Publishing Company, Easton, Pa., 1985, p. 1418 and Berge, SM et al, Journal of Pharmaceutical Science, 1977, 66, 1, 1-19. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Rifamycin analog compounds depicted herein include all isomeric (*e.g*., enantiomeric, diastereomeric, and geometric (or conformational)) forms of the compound; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the disclosure. All tautomeric forms of the compounds presented herein are also within the scope of the disclosure.

Rifamycin analog compounds described herein also include all compounds that differ only in the presence of one or more isotopically enriched atoms. For example, compounds having the present structures except for the replacement of hydrogen by deuterium or tritium, or the replacement of a carbon by a ¹¹C- or ¹³C- or ¹⁴C -enriched carbon, or the replacement of an oxygen by a ¹⁷O- or ¹⁸O-enriched oxygen, or the replacement of a nitrogen by a ¹⁵N-enriched nitrogen are within the scope of this disclosure.

Crystalline forms of the compounds of the disclosure and salts thereof are also within the scope of the disclosure. The compounds of the disclosure may be isolated in various amorphous and crystalline polymorphic forms, including without limitation amorphous and crystalline polymorphic forms which are anhydrous, hydrated, non-solvated, or solvated. Example hydrates include hemihydrates, monohydrates, dihydrates, and the like. In some embodiments, the compounds of the disclosure are anhydrous and non-solvated. By "anhydrous" is meant that the crystalline form of the compound contains essentially no bound water in the crystal lattice structure, i.e., the compound does not form a crystalline hydrate.

### Pharmaceutical Compositions and Dosage Forms

The present disclosure also provides pharmaceutical compositions comprising the rifamycin analog compounds described herein and vancomycin. When employed as pharmaceuticals, the rifamycin analogs and vancomycin can be administered in the form of pharmaceutical compositions which is a combination of rifamycin analogs and vancomycin and a pharmaceutically acceptable carrier. These compositions can be prepared in a manner well known in the pharmaceutical art, and can be administered by a variety of routes. Such pharmaceutical compositions can be administered systemically. The term "systemic" as used herein includes parenteral, topical, transdermal, oral, by inhalation/pulmonary, rectal, nasal, buccal, and sublingual administration. The term "parenteral" as used herein includes subcutaneous, intradermal, intravenous, intramuscular, intracranial, and intraperitoneal administration. In some embodiments, the compounds are administered orally, topically, intranasally, intravenously, intramuscularly, or subcutaneously in therapeutically effective amounts to treat bacterial infections (e.g., *S*. *aureus* infections).

Pharmaceutical compositions containing rifamycin analogs and vancomycin can be prepared in combination with one or more pharmaceutically acceptable carriers. In making the compositions of the disclosure, the active ingredient is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10 % by weight of the active compound, soft and hard gelatin capsules, suppositories, sterile injectable solutions, and sterile packaged powders.

In some embodiments, the pharmaceutical composition of the disclosure is in liquid form. Liquid forms include, by way of non-limiting example, emulsions, solutions, suspensions, syrups, slurries, dispersions, colloids and the like. In some embodiments, a pharmaceutical composition described herein is in liquid, semi-solid or solid (*e.g*., powder) form. In specific embodiments, a pharmaceutical composition described herein is in semi-solid form, *e.g*., a gel, a gel matrix, a cream, a paste, or the like. In some embodiments, semi-solid forms comprise a liquid vehicle. In some embodiments, the pharmaceutical composition of the disclosure is a solid dosage form, such a tablet, a granule, a sachet, or a powder. Also provided are pharmaceutical compositions comprising a compound of the disclosure or a pharmaceutically acceptable salt thereof in the form of a dissolving tablet, a dissolving wafer, a capsule, or a gel capsule. In certain embodiments, solid dosage forms described herein comprise a solid vehicle (*e.g*., as used in a tablet), and/or a gaseous vehicle (*e.g*., as used in DPI).

In some embodiments, a composition is in a unit dose formulation for oral, intranasal, intravenous, or other administration to a patient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The rifamycin analogs according to the disclosure in combination with vancomycin can be effective over a wide dosage range and are generally administered in a pharmaceutically effective amount. It will be understood, however, that the amount of the compound and/or combination of compounds actually administered will usually be determined by a physician, according to the relevant circumstances, including the condition to be treated, the chosen route of administration, the actual compound administered, the age, weight, and response of the individual patient, the severity of the patient's symptoms, and the like.

In some embodiments, a composition or unit dosage form described herein is administered as an emulsion, a solution, a suspension, a syrup, a slurry, a dispersion, a colloid, a dissolving tablet, a dissolving wafer, a capsule, a gel capsule, a semi-solid, a solid forma gel, a gel matrix, a cream, a paste, a tablet, a granule, a sachet, a powder, or the like.

In certain aspects, about 0.000001 mg to about 10000 mg, about 0.00001 mg to about 5000 mg, about 0.0001 mg to about 1000 mg, about 0.005 mg to about 750 mg, about 0.001 mg to about 500 mg, about 0.01 mg to about 300 mg, about 1 mg to about 200 mg, about 5 mg to about 500 mg, about 10 mg to about 750 mg, about 50 mg to about 1000 mg, about 100 mg to about 750 mg, about 125 mg to about 600 mg, or about 200 mg to about 500 mg, or about 50 mg, or about 75 mg, or about 100 mg, or about 150 mg, or about 200 mg, or about 250 mg, or about 300 mg, or about 400 mg, or about 500 mg, or about 600 mg, or about 700 mg, or about 750 mg, or about 1000 mg of the rifamycin analog (I) or (II) as provided herein, per day or per dose is administered to an individual.

In certain aspects, about 0.0001 mg/kg to about 10000 mg/kg, about 0.001 mg/kg to about 5000 mg/kg, about 0.01 mg/kg to about 1000 mg/kg, about 0.1 mg/kg to about 750 mg/kg, or about 0.125 mg/kg to about 600 mg/kg, or about 0.25 mg/kg to about 300 mg/kg, or about 0.5 mg/kg to about 100 mg/kg, or about 1 mg/kg to about 50 mg/kg, or about 0.001 mg/kg to about 50 mg/kg of, or about 0.002 mg/kg to about 25 mg/kg, or about 0.01 mg/kg to about 25 mg/kg, or about 0.02 mg/kg to about 25 mg/kg, or about 0.05 mg/kg to about 25 mg/kg, or about 0.01 mg/kg to about 25 mg/kg, or about 0.25 mg/kg to about 25 mg/kg of a rifamycin analog (I) or (II) per day or per dose is administered to an individual.

In certain aspects, about 0.001 mg to about 10000 mg, about 0.1 mg to about 5000 mg, about 1 mg to about 3000 mg, about 10 mg to about 2000 mg, about 100 mg to about 1000 mg, about 250 mg to about 750 mg, or about 100 mg, or about 200 mg, or about 250 mg, or about 300 mg, or about 400 mg, or about 500 mg, or about 600 mg, or about 700 mg, or about 750 mg or about 1000 mg, or about 1500 mg, or about 2000 mg of vancomycin or a derivative thereof, per day or per dose is administered to an individual.

In certain aspects, 0.0001 mg/kg to about 10000 mg/kg, about 0.001 mg/kg to about 5000 mg/kg, about 0.01 mg/kg to about 1000 mg/kg, about 0.1 mg/kg to about 750 mg/kg, or about 0.125 mg/kg to about 600 mg/kg, or about 0.25 mg/kg to about 300 mg/kg, or about 0.5 mg/kg to about 100 mg/kg, or about 1 mg/kg to about 50 mg/kg, or about 0.001 mg/kg to about 50 mg/kg of, or about 0.002 mg/kg to about 25 mg/kg, or about 0.01 mg/kg to about 25 mg/kg, or about 0.02 mg/kg to about 25 mg/kg, or about 0.05 mg/kg to about 25 mg/kg, or about 0.01 mg/kg to about 25 mg/kg, or about 0.25 mg/kg to about 25 mg/kg of vancomycin or a derivative thereof per day or per dose is administered to an individual.

In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g. about 40 mesh. Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the disclosure can be formulated so as to provide quick, sustained or delayed release of the active ingredient after administration to the patient by employing procedures known in the art.

For preparing solid compositions such as tablets, the principal active ingredient is mixed with a pharmaceutical excipient to form a solid pre-formulation composition containing a homogeneous mixture of the rifamycin analog and vancomycin. When referring to these pre-formulation compositions as homogeneous, each active ingredient is typically dispersed evenly throughout the composition so that the composition can be readily subdivided into equally effective unit dosage forms such as tablets, pills and capsules. This solid pre-formulation is then subdivided into unit dosage forms of the type described above containing from, for example, 0.001 to about 5000 mg of each active ingredient of the present application.

The tablets or pills containing the rifamycin analog and vancomycin can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the rifamycin analog and vancomycin and compositions comprising rifamycin analog and vancomycin of the present application can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face masks tent, or intermittent positive pressure breathing machine. Solution, suspension, or powder compositions can be administered orally or nasally from devices which deliver the formulation in an appropriate manner.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9. It will be understood that use of certain of the foregoing excipients, carriers, or stabilizers will result in the formation of pharmaceutical salts.

The therapeutic dosage of the compounds of the disclosure, i.e., rifamycin analogs and vancomycin, can vary according to, for example, the particular use for which the treatment is made, the manner of administration of the compound, the health and condition of the patient, and the judgment of the prescribing physician. The proportion or concentration of the compounds of the disclosure in a pharmaceutical composition can vary depending upon a number of factors including dosage, chemical characteristics (e.g., hydrophobicity), and the route of administration. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from in vitro or animal model test systems.

### Kits

The present application also includes pharmaceutical kits useful, for example, in the treatment of bacterial infections (e.g., S. *aureus* infections), which include one or more containers containing a pharmaceutical composition comprising a therapeutically effective amount of a rifamycin analog and vancomycin. Such kits can further include, if desired, one or more of various conventional pharmaceutical kit components, such as, for example, containers with one or more pharmaceutically acceptable carriers, additional containers, etc., as will be readily apparent to those skilled in the art. Instructions, either as inserts or as labels, indicating quantities of the components to be administered, guidelines for administration, and/or guidelines for mixing the components, can also be included in the kit.

In one embodiment, a kit may comprise the rifamycin analog compound and the vancomycin in the same container.

In another embodiment, , a kit may comprise the rifamycin analog compound and the vancomycin in separate containers.

In one embodiment, the present disclosure provides a kit comprising a) a first container comprising a compound selected from 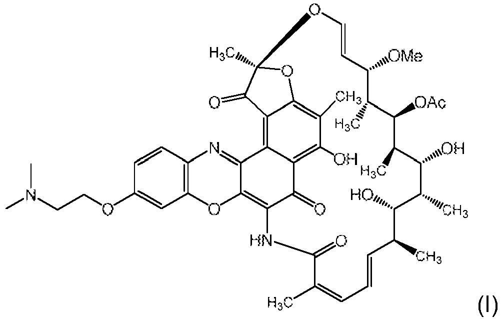 and 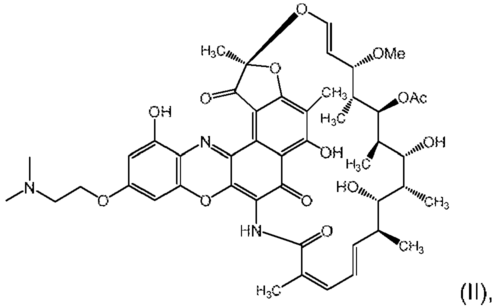 or a pharmaceutically acceptable salt thereof,
b) a second container comprising vancomycin or an ester, amide, N-oxide, a pharmaceutically acceptable salt of an ester or amide, or a pharmaceutically acceptable salt thereof, and
c) instructions for administration of the compound (I) or (II) and the vancomycin.

Delivery devices are important not only for delivering the compounds of the disclosure, but also for providing an appropriate environment for storage. This would include protection from microbial contamination and chemical degradation. The device and formulation should be compatible so as to avoid potential leaching or adsorption. The delivery device (or its packaging) can be optionally provided with a label and/or with instructions for use indicating how the composition(s) should be used.

### Methods of Use

In another aspect, the present disclosure provides a method of preventing or inhibiting growth of a bacterium comprising administering an effective amount of:
a) a compound selected from 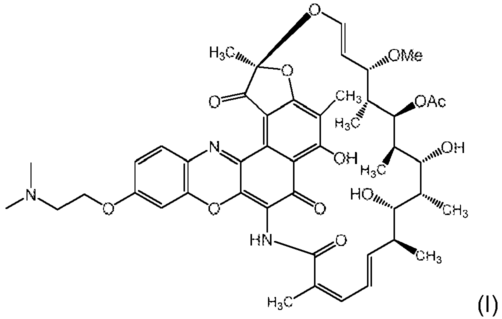 and 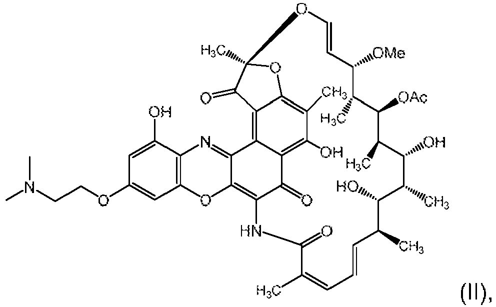 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of preventing or inhibiting growth of a bacterium comprising administering an effective amount of:
a) 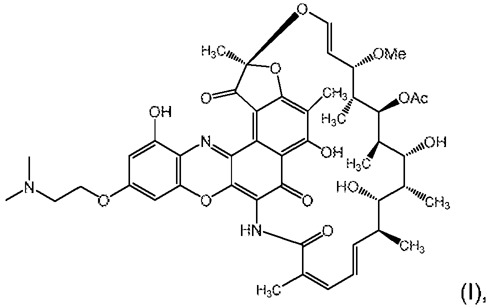 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of preventing or inhibiting growth of a bacterium comprising administering an effective amount of:
a) 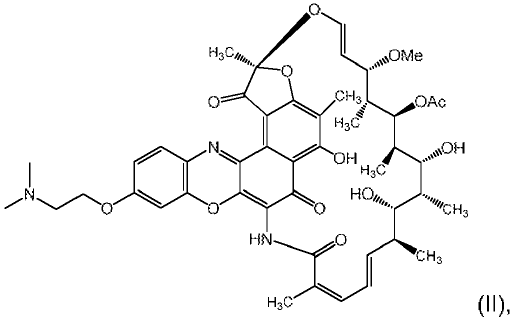 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of preventing or inhibiting growth of a bacterium comprising administering an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is a Gram-positive bacterium.

In one embodiment, the present disclosure provides a method of preventing or inhibiting growth of a bacterium comprising administering an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is a penicillin-resistant bacterium, or a methicillin-resistant bacterium.

In one embodiment, the present disclosure provides a method of preventing or inhibiting growth of a bacterium comprising administering an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is *Staphylococcus aureus,* e.g., methicillin-resistant *Staphylococcus aureus* (MRSA) and methicillin-susceptible *Staphylococcus aureus* (MSSA).

In another aspect, the present disclosure provides a method of treating a bacterial infection in a subject in need of such treatment comprising administering to the subject an effective amount of:
a) a compound selected from 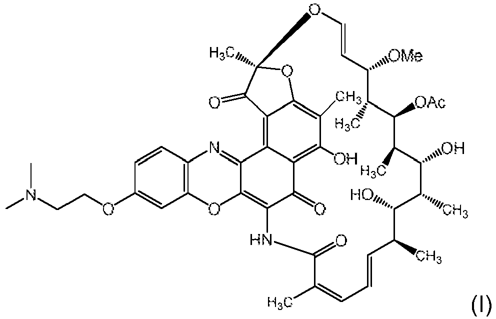 and 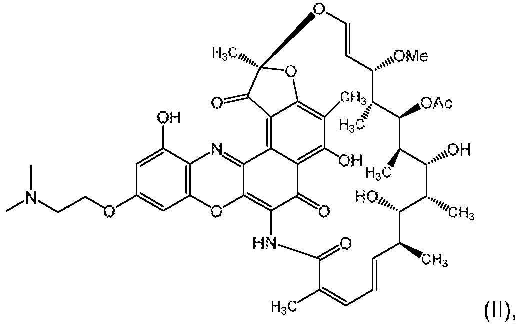 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of treating a bacterial infection in a subject in need of such treatment comprising administering to the subject an effective amount of:
a) 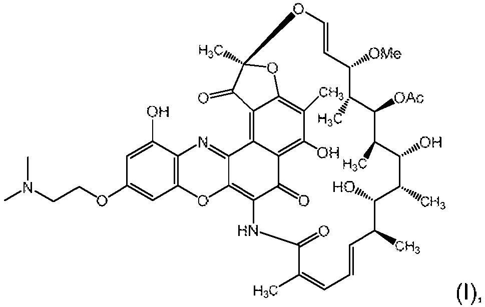 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of treating a bacterial infection in a subject in need of such treatment comprising administering to the subject an effective amount of:
a) 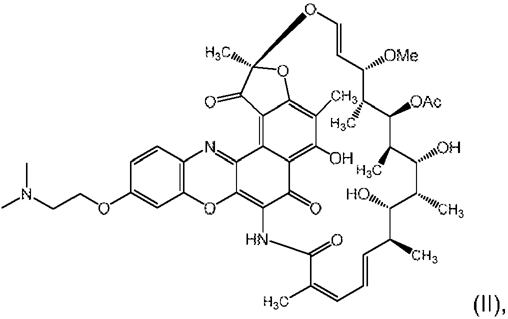 or a pharmaceutically acceptable salt thereof, and
b) vancomycin or an ester, amide, N-oxide, a pharmaceutically acceptable salt of an ester or amide, or a pharmaceutically acceptable salt thereof.

In one embodiment, the present disclosure provides a method of treating a bacterial infection in a subject in need of such treatment comprising administering to the subject an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is a Gram-positive bacterium.

In one embodiment, the present disclosure provides a method of treating a bacterial infection in a subject in need of such treatment comprising administering to the subject an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is a penicillin-resistant bacterium, or a methicillin-resistant bacterium.

In one embodiment, the present disclosure provides a method of treating a bacterial infection in a subject in need of such treatment comprising administering to the subject an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterium is *Staphylococcus aureus,* e.g., methicillin-resistant *Staphylococcus aureus* (MRSA) and methicillin-susceptible *Staphylococcus aureus* (MSSA).

In one embodiment, the present disclosure provides a method of treating a bacterial infection in a subject in need of such treatment comprising administering to the subject an effective amount of a) a compound (I) or (II) or a pharmaceutically acceptable salt thereof, and b) vancomycin or an ester, amide, N-oxide, a pharmaceutically acceptable salt of an ester or amide, or a pharmaceutically acceptable salt thereof, wherein the bacterial infection is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

In one aspect, the present disclosure provides a method of preventing or treating a disease in a subject in need thereof comprising administering to the subject an effective amount of a) a compound selected from 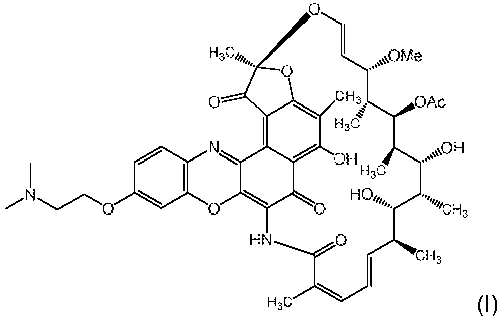 and 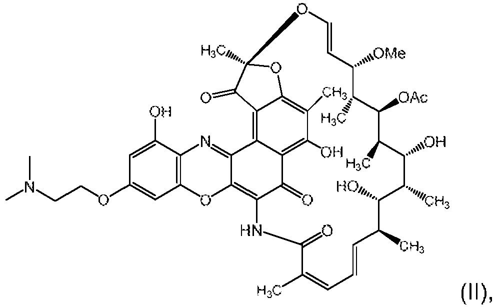 or a pharmaceutically acceptable salt thereof, and
b) vancomycin or an ester, amide, N-oxide, a pharmaceutically acceptable salt of an ester or amide, or a pharmaceutically acceptable salt thereof,
wherein the disease is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

In one aspect, the present disclosure provides a method of enhancing efficacy of vancomycin or an ester, amide, N-oxide, a pharmaceutically acceptable salt of an ester or amide thereof comprising administering to a subject in need thereof an effective amount of:
a) a compound selected from 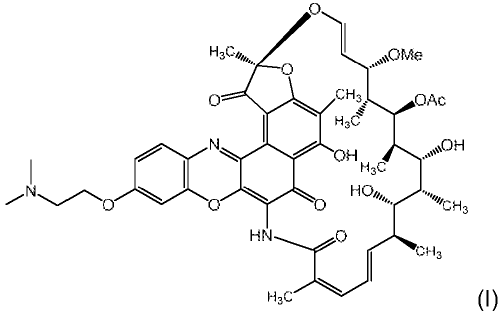 and 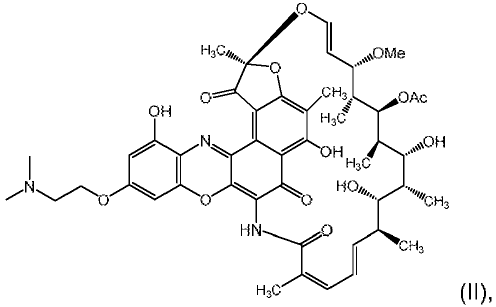 or a pharmaceutically acceptable salt thereof, in combination with
b) vancomycin or an ester, amide, N-oxide, a pharmaceutically acceptable salt of an ester or amide, or a pharmaceutically acceptable salt thereof.

In one aspect, the present disclosure provides a method of enhancing efficacy of a compound selected from 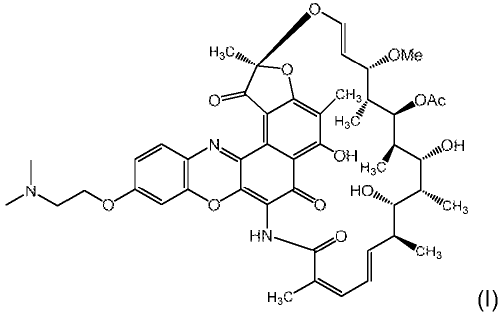 and 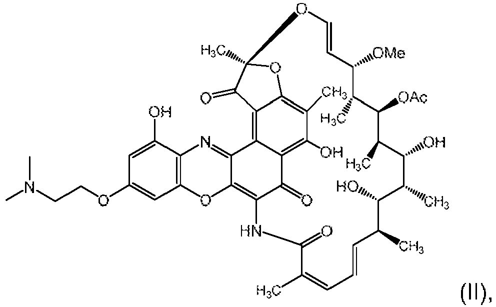 or a pharmaceutically acceptable salt thereof, the method comprising administering a subject in need thereof an effective amount of the compound (I) or (II) in combination with vancomycin or an ester, amide, N-oxide, a pharmaceutically acceptable salt of an ester or amide, or a pharmaceutically acceptable salt thereof.

In one embodiment of any of the methods described herein, the compound (I) or (II) and vancomycin can be administered systemically. The term "systemic" as used herein includes parenteral, topical, transdermal, oral, by inhalation/pulmonary, rectal, nasal, buccal, and sublingual administration. The term "parenteral" as used herein includes subcutaneous, intradermal, intravenous, intramuscular, intracranial, and intraperitoneal administration.

In some embodiments of any of the methods described herein, the compounds (i.e., compound (I) or (II) and vancomycin) are administered orally, topically, intranasally, intravenously, intramuscularly, or subcutaneously in therapeutically effective amounts to treat bacterial infections (e.g., S. aureus infections).

In one embodiment of any of the methods described herein, one or both compounds are administered orally.

In another embodiment of any of the methods described herein, one or both compounds are administered parenterally, i.e., intravenously, intramuscularly, or subcutaneously.

In another embodiment of any of the methods described herein, one or both compounds are administered topically.

In another embodiment of any of the methods described herein, one or both compounds are administered intranasally.

In one embodiment of any of the methods described herein, the subject is a mammal. In one embodiment of any of the methods described herein, the subject is human.

In one embodiment of any of the methods described herein, the method comprises administering a third therapeutic agent. In one embodiment, the third therapeutic agent is a third antibiotic. In one embodiment, the third antibiotic is effective against Staphylococcus aureus.

In one embodiment, the third antibiotic is selected from an aminoglycoside, a beta-lactam, a macrolide, a cyclic peptide, a tetracycline, a fluoroquinoline, a fluoroquinolone, and an oxazolidinone.

In one embodiment, the third antibiotic is selected from clindamycin, novobiocin, retapamulin, daptomycin, sitafloxacin, teicoplanin, triclosan, napthyridone, radezolid, doxorubicin, ampicillin, imipenem, doripenem, gemcitabine, dalbavancin, and azithromycin.

In one embodiment of any of the above methods, the method comprises administering a pharmaceutical composition comprising a rifamycin analog and vancomycin, or a pharmaceutically acceptable salt thereof, or a pharmaceutical dosage form comprising a rifamycin analog and vancomycin, or a pharmaceutically acceptable salt thereof of the present disclosure.

In one embodiment, the composition, or the dosage form is administered to the subject orally, topically, intranasally, intravenously, intramuscularly, or subcutaneously.

In one embodiment of any of the above methods, the compound (I) or (II), or a pharmaceutically acceptable salt thereof, and the vancomycin or a derivative thereof, as defined in the claims, or a pharmaceutically acceptable salt thereof, are administered in a single dosage form. This dosage form may be a solid or a liquid dosage form comprising both active agents.

In another embodiment of any of the above methods, the compound (I) or (II), or a pharmaceutically acceptable salt thereof, and the vancomycin or a derivative thereof, as defined in the claims, or a pharmaceutically acceptable salt thereof, are administered in separate dosage forms.

The separate dosage forms may both be solid dosage forms, or liquid dosage forms, or one liquid and one solid dosage forms.

In one embodiment, the dosage form comprising the rifamycin analog (I) or (II) is a dosage form for oral administration, and the dosage form comprising vancomycin is a dosage form for intravenous or subcutaneous administration.

In one embodiment, the dosage form comprising the rifamycin analog (I) or (II) is a dosage form for oral administration, and the dosage form comprising vancomycin is a dosage form for intravenous administration.

In another embodiment, both dosage forms are for intravenous or subcutaneous administration.

In yet another embodiment, both dosage forms are for oral administration.

The separate dosage forms may be administered simultaneously. In some embodiments, the separate dosage forms may be administered simultaneously on the same day, or within 30 min from each other, or within 1 hour from each other.

The separate dosage forms may be administered at different times. When the separate dosage forms are administered at different times, they may be administered on the same day or on separate days, e.g., consecutive days or non-consecutive days. In some embodiments, the separate dosage forms may be administered at separate times on the same day. In some embodiments, the separate dosage forms may be administered at least 1 day apart, or at least 2 days apart, or at least 3 days apart, or at least 4 days apart.

In one embodiment of any of the above methods, the compound (I) or (II), or a pharmaceutically acceptable salt thereof, and the vancomycin or a derivative thereof, as defined in the claims, or a pharmaceutically acceptable salt thereof, are administered on the same day. In another embodiment of any of the above methods, the compound (I) or (II), or a pharmaceutically acceptable salt thereof, and the vancomycin or a derivative thereof, as defined in the claims, or a pharmaceutically acceptable salt thereof, are administered on the same day in separate dosage forms.

In another embodiment of any of the above methods, the compound (I) or (II), or a pharmaceutically acceptable salt thereof, and the vancomycin or a derivative thereof, as defined in the claims, or a pharmaceutically acceptable salt thereof, are administered simultaneously. In another embodiment of any of the above methods, the compound (I) or (II), or a pharmaceutically acceptable salt thereof, and the vancomycin or a derivative thereof, as defined in the claims, or a pharmaceutically acceptable salt thereof, are administered simultaneously in separate dosage forms.

In another embodiment of any of the above methods, the compound (I) or (II), or a pharmaceutically acceptable salt thereof, and the vancomycin or a derivative thereof, as defined in the claims, or a pharmaceutically acceptable salt thereof, are not administered on the same day.

In another embodiment of any of the above methods, the compound (I) or (II), or a pharmaceutically acceptable salt thereof, and the vancomycin or a derivative thereof, as defined in the claims, or a pharmaceutically acceptable salt thereof, are administered on consecutive days.

In another embodiment of any of the above methods, the compound (I) or (II), or a pharmaceutically acceptable salt thereof, and the vancomycin or a derivative thereof, as defined in the claims, or a pharmaceutically acceptable salt thereof, are administered on separate, non-consecutive days.

In another embodiment of any of the above methods, the vancomycin or a derivative thereof, as defined in the claims, or a pharmaceutically acceptable salt thereof, is administered twice daily.

In another embodiment of any of the above methods, the compound (I) or (II), or a pharmaceutically acceptable salt thereof, is administered once daily.

In certain aspects of any of the above methods, about 0.000001 mg to about 10000 mg, about 0.00001 mg to about 5000 mg, about 0.0001 mg to about 1000 mg, about 0.005 mg to about 750 mg, about 0.001 mg to about 500 mg, about 0.01 mg to about 300 mg, about 1 mg to about 200 mg, about 5 mg to about 500 mg, about 10 mg to about 750 mg, about 50 mg to about 1000 mg, about 100 mg to about 750 mg, about 125 mg to about 600 mg, or about 200 mg to about 500 mg, or about 50 mg, or about 75 mg, or about 100 mg, or about 150 mg, or about 200 mg, or about 250 mg, or about 300 mg, or about 400 mg, or about 500 mg, or about 600 mg, or about 700 mg, or about 750 mg, or about 1000 mg of the rifamycin analog (I) or (II) as provided herein, per day or per dose is administered to an individual.

In certain aspects of any of the above methods, about 0.0001 mg/kg to about 10000 mg/kg, about 0.001 mg/kg to about 5000 mg/kg, about 0.01 mg/kg to about 1000 mg/kg, about 0.1 mg/kg to about 750 mg/kg, or about 0.125 mg/kg to about 600 mg/kg, or about 0.25 mg/kg to about 300 mg/kg, or about 0.5 mg/kg to about 100 mg/kg, or about 1 mg/kg to about 50 mg/kg, or about 0.001 mg/kg to about 50 mg/kg of, or about 0.002 mg/kg to about 25 mg/kg, or about 0.01 mg/kg to about 25 mg/kg, or about 0.02 mg/kg to about 25 mg/kg, or about 0.05 mg/kg to about 25 mg/kg, or about 0.01 mg/kg to about 25 mg/kg, or about 0.25 mg/kg to about 25 mg/kg of a rifamycin analog (I) or (II) per day or per dose is administered to an individual.

In certain aspects of any of the above methods, about 0.001 mg to about 10000 mg, about 0.1 mg to about 5000 mg, about 1 mg to about 3000 mg, about 10 mg to about 2000 mg, about 100 mg to about 1000 mg, about 250 mg to about 750 mg, or about 100 mg, or about 200 mg, or about 250 mg, or about 300 mg, or about 400 mg, or about 500 mg, or about 600 mg, or about 700 mg, or about 750 mg or about 1000 mg, or about 1500 mg, or about 2000 mg of vancomycin or a derivative thereof, as defined in the claims, per day or per dose is administered to an individual.

In certain aspects of any of the above methods, 0.0001 mg/kg to about 10000 mg/kg, about 0.001 mg/kg to about 5000 mg/kg, about 0.01 mg/kg to about 1000 mg/kg, about 0.1 mg/kg to about 750 mg/kg, or about 0.125 mg/kg to about 600 mg/kg, or about 0.25 mg/kg to about 300 mg/kg, or about 0.5 mg/kg to about 100 mg/kg, or about 1 mg/kg to about 50 mg/kg, or about 0.001 mg/kg to about 50 mg/kg of, or about 0.002 mg/kg to about 25 mg/kg, or about 0.01 mg/kg to about 25 mg/kg, or about 0.02 mg/kg to about 25 mg/kg, or about 0.05 mg/kg to about 25 mg/kg, or about 0.01 mg/kg to about 25 mg/kg, or about 0.25 mg/kg to about 25 mg/kg of vancomycin or a derivative thereof, as defined in the claims, per day or per dose is administered to an individual.

### EXAMPLES

The following examples illustrate specific aspects of the instant description. The examples should not be construed as limiting, as the examples merely provide specific understanding and practice of the embodiments and their various aspects.

As used herein, the symbols and conventions used in the processes, and Examples, herein, are consistent with those used in the contemporary scientific literature, for example, the Journal of the American Chemical Society or the Journal of Biological Chemistry unless specified otherwise to the contrary. Only the presently claimed compounds form part of the present invention. Other compounds are used as reference examples, only.

### Example 1: Synthesis of Rifamycin Analog (I)

Rifamycin analog (I) was synthesized from rifamycin S as shown in Scheme 1, below, and as described below.

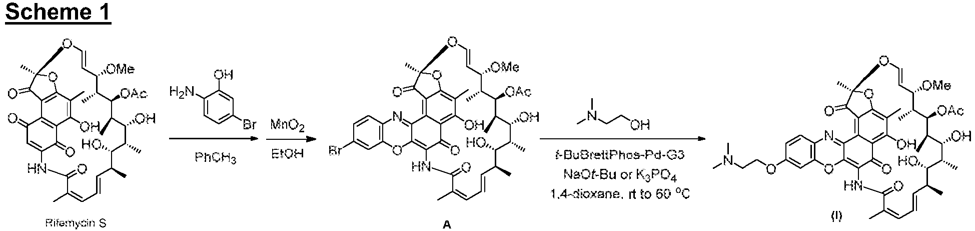

*Synthesis of Intermediate A.* To a stirring solution under argon of rifamycin S (2.0 g, 2.87 mmol) in 80 mL of toluene at room temperature was added 2-amino-5-bromophenol (0.54 g, 2.87 mmol). The solution was stirred for 2 days at room temperature. The reaction mixture was then evaporated to dryness and the dark residue dissolved in 20 mL of ethanol and 300 mg of manganese oxide (MnO₂) was added in one portion to the ethanol solution. The sluggish mixture was stirred under argon for 15 h at room temperature. After filtration of insoluble materials using a Celite pad, the filtrate was evaporated under reduced pressure. The dark residue was purified on a 120 g HP silica gel Gold RediSep column *via* ISCO (gradient elution: 5 → 95% EA in hexanes). The pure fractions were evaporated and dried *in vacuo* giving the title Intermediate A as a dark reddish solid (1.6 g, 65%). MS (ESI, pos.): calc'd for C₄₃H₄₇BrN₂O₁₃, 862.23; found 863.1 and 865.1 (M+H), 885.1 and 888.0 (M+Na). ¹H NMR (500 MHz; DMSO-d₆): δ 9.49 (d, *J* = 6.0 Hz, 1H), 7.92 (ddd, *J* = 3.6, 2.9, 1.8 Hz, 1H), 7.86 - 7.85 (m, 1H), 7.75 - 7.74 (m, 1H), 6.06 - 6.05 (m, 1H), 5.84 (dt, *J* = 2.6, 1.4 Hz, 2H), 5.25 - 5.23 (m, 2H), 4.80 (dt, *J* = 2.5, 1.0 Hz, 1H), 4.23 (td, *J* = 2.4, 1.0 Hz, 1H), 3.49 (d, *J* = 1.1 Hz, 1H), 3.10 - 3.09 (m, 2H), 3.03 (s, 3H), 2.79 (s, 1H), 2.19 (s, 3H), 2.01 (s, 4H), 1.96 (s, 4H), 1.81 (d, *J* = 2.2 Hz, 1H), 1.68 (s, 3H), 1.60 (dq, *J* = 2.8, 0.9 Hz, 1H), 1.48 (t, *J* = 1.4 Hz, 1H), 0.90 (dt, *J* = 2.1, 1.1 Hz, 2H), 0.84 (d, *J* = 7.1 Hz, 4H), 0.69 (dd, *J* = 2.2, 1.2 Hz, 5H).

*Synthesis of rifamycin analog (I).* Using a similar method reported by Buchwald S. L. et al. (Org. Lett. 2018, 20, 1580), a palladium-catalyzed C-O coupling of primary alcohols with Intermediate A was employed for title rifamycin analog (I). To a 2 dram screw-top oven-dried test tube, equipped with a stir bar, and sealed with a screw cap was charged Intermediate A (40 mg, 0.0463 mmol, 1.00 eq.), 2-(dimethylamino)ethan-1-ol (42 mg, 0.462 mmol, 10 eq.), tBuBrettPhos Pd G3-palladacycle (11.8 mg, 30 mol%), and NaOt-Bu (5 mg, 0.051 mmol, 1.1 eq.). The reaction tube was recapped with a septum and pierced with a needle attached to evacuate and backfilled with argon (this process was repeated twice) followed by addition of 1,4-dioxane (2.0 mL) via syringe. The reaction was heated at 55°C ± 5 in an oil bath under argon pressure for 15 h, the reaction was allowed to cool to room temperature before filtration through a pad of Celite^{®} and rinsed with EtOAc. The crude material was concentrated in vacuo and purified on a 15.5 g C18 Aq Gold column (gradient elution: 10 - 95% MeCN in water, 0.05% acetic acid in both). The product fractions were combined, frozen on dry ice, and lyophilized giving the title rifamycin analog (I) as a dark reddish solid (12.5 mg, 32%). MS: calc'd for C₄₇H₅₇N₃O₁₃, 871.39; found 872.3 (M+H), 870.2 (M-H). ¹H NMR (300 MHz; DMSO-d₆) *δ* 9.40 (s, 1H), 7.87 (d, *J =* 8.9 Hz, 1H), 7.23-7.16 (m, 2H), 6.83 (dt, *J* = 2.3, 1.1 Hz, 1H), 6.23 (d, *J* = 4.6 Hz, 1H), 6.06 (dd, *J* = 5.9, 1.1 Hz, 1H), 5.82 (dd, *J* = 2.3, 1.5 Hz, 2H), 5.24 (dt, *J* = 1.4, 0.7 Hz, 1H), 4.83-4.75 (m, 1H), 4.24 (d, *J = 29.9* Hz, 3H), 3.80 (d, *J* = 1.3 Hz, 1H), 3.03 (t, *J* = 0.5 Hz, 3H), 2.88 (s, 1H), 2.78 (t, *J = 0.9* Hz, 2H), 2.67 (s, 2H), 2.22 (d, *J =* 3.7 Hz, 4H), 2.15 (s, 2H), 2.02 (s, 2H), 1.96 (d, *J* = 1.2 Hz, 2H), 1.90 (s, 1H), 1.68 (s, 2H), 0.85 (d, *J* = 6.7 Hz, 3H), 0.69 (t, *J* = 1.2 Hz, 3H).

### Example 2: Synthesis of Rifamycin Analog (II)

Rifamycin analog (II) was prepared as shown in Scheme 2, below, and described below.

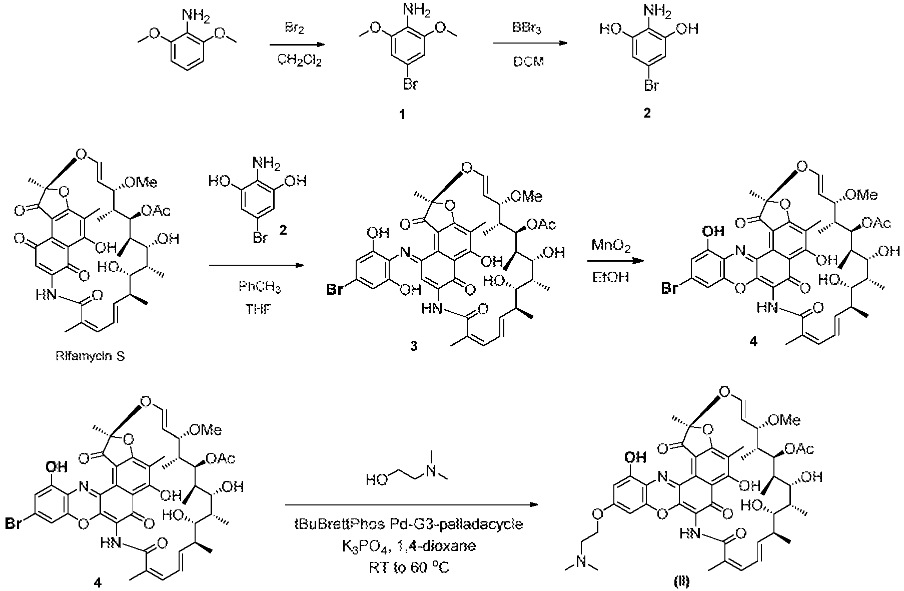

*Synthesis of Compound 1.* To a stirred solution of 2,6-dimethoxyaniline (9.0 g, 58.7 mmol, 1.0 eq) in 350 mL of anhydrous DCM was dropwise added over 30 min a Br₂ solution in 50 mL of anhydrous DCM at 4° C. An additional 200 mL was added to the slurry to achieve a semi-homogeneous solution. The reaction mixture was stirred overnight at room temperature. The dark brown mixture was cooled to 4° C and basified by addition of 1.0 M NaOH solution (ca. *100* mL) to pH = 10-11. The mixture was diluted with 200 mL of DCM and the layers are separated. The aqueous layer was extracted with DCM (200 mL total). The combined DCM layers were washed with water, brine, and dried over Na₂SO₄. After concentration *in vacuo,* the crude product was obtained as a slightly reddish solid. The residue was dissolved in DCM (8 mL) and loaded onto a 220 g HP silica gel Gold RediSep column and purified *via* ISCO (gradient elution: 5 - 95% EA in hexanes), pure fractions combined, and the solvent evaporated *in vacuo.* The solid was triturated with DCM and hexanes and filtered. The off-while solid was dried *in vacuo* giving the title compound 1 as an off-white solid (9.4 g, 70%). MS (ESI, pos.): calc'd for C₈H₁₀BrNO₂, 230.99; found 231.9 and 234.0 (M+H). ¹H NMR (500 MHz; CDCl₃) *δ* 6.66 (s, 2H), 3.84 (s, 6H).

*Synthesis of Compound 2.* Compound 1 (2.2 g, 9.47 mmol, 1.0 eq) was dissolved in 10 mL of anhydrous DCM and a BBr₃ solution was added dropwise over 10 min (10 mL, 1.0 M solution in DCM) at 4° C. The reaction was exothermic and produced a precipitate. An additional amount of BBr₃ (9 mL, 94.7 mmol, 10 eq) was added and the reaction mixture was stirred at room temperature overnight. The reddish suspension was checked by LC/MS to confirm the desired product. The reaction mixture was transferred to a 250 mL flask and cooled to 4° C. The mixture was carefully quenched with water followed by treatment with aqueous saturated NaHCO₃ solution to give a pH = 7-8. The mixture was extracted with DCM and the aqueous layer cooled to 4° C to afford a dark brown precipitate. The mixture was filtered and the brown solid was dissolved in 10 mL of methanol and dried over Na₂SO₄ to provide the desired product 2 (1.9 g, 100%). MS (ESI, pos.): calc'd for C₆H₆BrNO₂, 202.96; found 204.0 and 206.1 (M+H). ¹H NMR (500 MHz; CD₃OD) *δ* 6.45 (s, 2H), 4.87 (s, 2H).

*Synthesis of Compound 4.* To a stirred solution of compound 2 (0.146 g, 0.72 mmol) in a mixture of toluene (20 mL) and THF (20 mL) at room temperature was added rifamycin S (0.5 g, 0.72 mmol). The solution was stirred for 3 days at room temperature to afford the desired product 3. The solvents were removed *in vacuo,* the dark residue was dissolved in 10 mL of ethanol followed by 100 mg of manganese dioxide (MnO₂). The sluggish mixture was stirred for 5 h at room temperature. After filtration of insoluble materials using a Celite pad, the filtrate was evaporated *in vacuo.* The dark residue was purified on a 120 g HP silica gel Gold RediSep column *via* ISCO (gradient elution: 5 - 95% EA in hexanes). The pure fractions combined and evaporated *in vacuo* giving the title compound 4 as a dark reddish solid (270 mg, 43%). MS (ESI, pos.): calc'd for C₄₃H₄₇BrN₂O₁₃, 878.23; found 879.2 and 880.2 (M+H), 878.1 and 879.1 (M-1). ¹H NMR (500 MHz; DMSO-d₆) *δ* 10.22 (br. s., 1H), 9.52 (br. s., 1H), 7.43 (br. s., 1H), 7.35 (br. s., 1H), 6.04 (br. s., 1H), 5.83 (br. s., 2H), 5.21 (d, *J* = 6.35 Hz, 2H), 4.89 (t, *J* = 10.50 Hz, 1H), 4.16 (br. s., 1H), 3.51 (br. s., 1H), 3.15 (br. s., 2H), 3.02 (br. s., 4H), 2.80 (t, *J =* 8.55 Hz, 1H), 2.21 (br. s., 3H), 2.08 (br. s., 1H), 1.96 (s, 4H), 1.99 (s, 4H), 1.78 (br. s., 1H), 1.71 (br. s., 3H), 1.60 (br. s., 1H), 1.47 (br. s., 1H), 0.84 (d, *J* = 6.84 Hz, 6H), 0.69 (br. s., 6H).

*Synthesis of Rifamycin Analog* (II). To a 8 mL screw-top oven-dried vial, equipped with a stir bar was charged with compound 4 (60 mg, 0.069 mmol, 1.00 eq), 2-(dimethylamino)ethan-1-ol (61 mg, 0.69 mmol, 10 eq), *t*-BuBrettPhos-Pd-G3-palladacycle (31 mg, 0.0345 mmol, 0.5 eq), and K₃PO₄ (30 mg, 0.141 mmol, 2.0 eq.). The reaction vial was capped with a rubber septum. The septum was pierced with a needle attached to evacuate and backfilled with argon (this process was repeated twice) followed by the addition of 1,4-dioxane (1.5 mL). The reaction was heated at 60 °C under argon pressure for 15 h, the reaction was allowed to cool to room temperature, filtered through a pad of Celite^{®}, and rinsed with MeOH. The crude material was concentrated *in vacuo* and purified on a 50 g C18 Aq column (gradient elution: 10 - 95% MeCN in water, 0.05% acetic acid in both). The product fractions were combined and lyophilized giving the title rifamycin analog (II) as a dark reddish solid (21 mg, 35%). MS (ESI, pos.): calc'd for C₄₇H₃₇N₃O₁₄, 887.38; found 888.3 (M+H). ¹H NMR (500 MHz; DMSO-d₆) *δ* 10.12 (br. s., 1H), 9.39 (br. s., 1H), 6.75 (br. s., 1H), 6.70 (br. s., 1H), 6.03 (br. s., 1H), 5.77 (d, *J =* 15.14 Hz, 1H), 5.21 (br. s., 1H), 4.83 - 4.90 (m, 1H), 4.15 - 4.30 (m, 2H), 4.08 (br. s., 1H), 3.53 (br. s., 1H), 3.29 (s, 1H), 3.16 (br. s., 1H), 3.03 (br. s., 3H), 2.87 (br. s., 1H), 2.79 (br. s., 1H), 2.62 - 2.71 (m, 2H), 2.36 (s, 1H), 2.23 (s, 6H), 2.19 (br. s., 3H), 1.93 - 2.11 (m, 7H), 1.91 (s, 1H), 1.76 (br. s., 1H), 1.69 (br. s., 3H), 1.53 - 1.65 (m, 1H), 1.50 (br. s., 1H), 1.32 - 1.45 (m, 1H), 0.76 - 0.94 (m, 6H), 0.68 (br. s., 5H).

### Example 3: S. aureus IV advanced disseminated infection mouse model

To test the efficacy of rifamycin analogs (I) and (II) according to the disclosure in combination therapy with vancomycin *in vivo,* a 4-day intravenous disseminated infection model was utilized. S. *aureus* MSRA strain NRS384 or N315, where indicated, was grown overnight in tryptic soy broth (TSB) and sub-cultured to mid-logarithmic phase. Bacteria were then washed twice with PBS and resuspended in PBS at a concentration of 1.5x10^8 cfu/mL NRS384 or 6.0x10^8 N315. Six-week-old Balb/c mice were then infected intravenously through the tail vein with 100 µL of the bacterial suspension, for a final infectious dose of 1.5x10^7 cfu/mouse NRS384 or 6.0x10^7 N315. From one to three days post infection, indicated mice were treated with 110 mg/kg vancomycin subcutaneously twice daily where indicated. Rifamycin analogs (I) or (II) were administered subcutaneously at the indicated dose two days after infection. Mice were monitored for weight loss and body conditioning score throughout the infection. At four days post infection, mice were euthanized, and the *S*. *aureus* kidney burden was quantified by tissue homogenization followed by enumeration of colony forming units through serial dilution in PBS and plating onto trypticase soy agar plates. Data points represent the kidney burden from individual mice tested.

### Example 3A: Median S. aureus NRS384 kidney burden in mice treated with rifampicin in combination with vancomycin

In this experiment, mice infected with *S*. *aureus* MSRA strain NRS384 were treated with vancomycin alone (control) or vancomycin in combination with 0.002 mg/kg to 25 mg/kg rifampicin, a commercially-available rifamycin analog control.

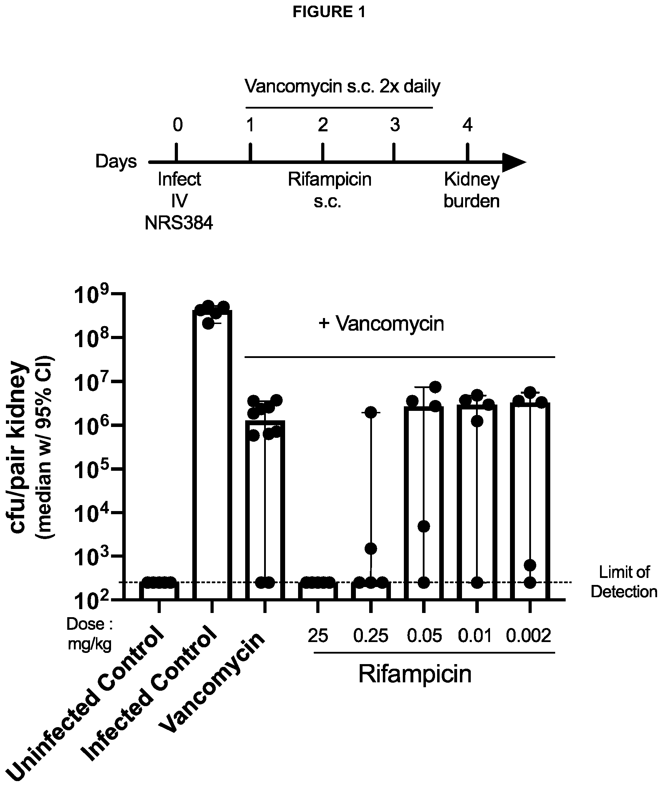

As shown in **Figure 1** and **Table 1,** intravenous infection with *S*. *aureus* MRSA strain NRS384 results in high bacterial burden in the kidneys. Vancomycin treatment alone results in merely a ~2-3 log reduction in S. aureus kidney burden. Combination treatment of rifampicin with vancomycin further reduces kidney burden at higher doses of rifampicin, as shown below.

**Table 1: Median S. aureus NRS384 kidney burden in mice treated with rifampicin in combination with vancomycin**

| **Treatment** | **Dose (mg/kg)** | **Vancomycin treatment** | **Median cfu/ kidney pair** | **Standard deviation** |
|---|---|---|---|---|
| Uninfected Control | | - | 2.50E+02 | 0.00E+00 |
| Infected Control | | - | 4.25E+08 | 1.25E+08 |
| Vancomycin Control | 110 mg/kg twice daily | + | 1.30E+06 | 1.43E+06 |
| Rifampicin | 25 | + | 2.50E+02 | 0.00E+00 |
| | 0.25 | + | 2.50E+02 | 8.94E+05 |
| | 0.05 | + | 2.75E+06 | 3.10E+06 |
| | 0.01 | + | 3.00E+06 | 1.95E+06 |
| | 0.002 | + | 3.38E+06 | 2.46E+06 |
| No mortality in study | | | Limit of detection = 250 | |

Rifampicin was effective in combination with vancomycin at 25 and 0.25 mg/kg, but efficacy was reduced at lower doses.

### Example 3B: Median S. aureus NRS384 kidney burden in mice treated with rifamycin analogs (I) or (II) in combination with vancomycin

In this experiment, mice infected with S. *aureus* MSRA strain NRS384 were treated with vancomycin alone (control) or vancomycin in combination with 0.01 - 0.25 mg/kg rifamycin analog (I) or 0.01 - 0.25 mg/kg rifamycin analog (II).

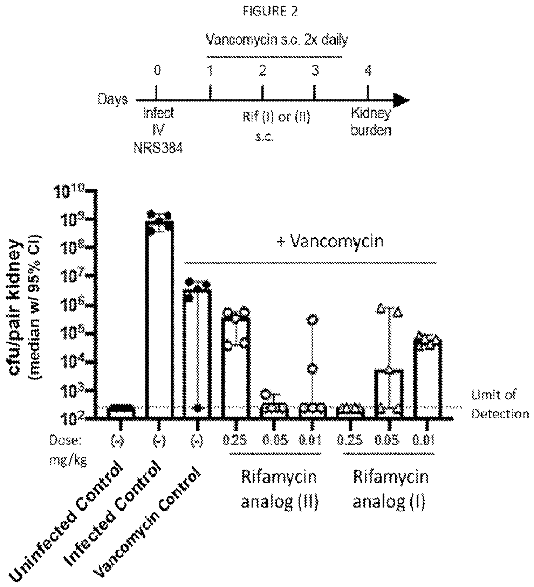

As shown in Figure 2 and Table 2, intravenous infection with S. *aureus* MRSA strain NRS384 results in high bacterial burden in the kidneys. Vancomycin treatment alone resulted in merely a ~2-3 log reduction in S. *aureus* kidney burden. Combination treatment of rifamycin analogs (I) and (II) with vancomycin further reduced kidney burden.

**Table 2: Median S. aureus NRS384 kidney burden in mice treated with rifamycin analogs (I) and (II) in combination with vancomycin**

| **Treatment** | **Dose (mg/kg)** | **Vancomycin treatment** | **Median cfu/ kidney pair** | **Standard deviation** |
|---|---|---|---|---|
| Uninfected Control | | - | 2.50E+02 | 0.00E+00 |
| Infected Control | | - | 8.88E+08 | 5.14E+08 |
| Vancomycin Control | 110 mg/kg twice daily | + | 3.50E+06 | 2.55E+06 |
| Rifamycin Analog (II) | 0.25 | + | 3.38E+05 | 2.51E+05 |
| | 0.05 | + | 2.50E+02 | 2.24E+02 |
| | 0.01 | + | 2.50E+02 | 1.33E+05 |
| Rifamycin Analog (I) | 0.25 | + | 2.50E+02 | 0.00E+00 |
| | 0.05 | + | 5.75E+03 | 3.86E+05 |
| | 0.01 | + | 5.88E+04 | 2.05E+04 |
| No mortality in study | | | Limit of detection = 250 | |

**Rifamycin** analogs (I) and (II) in combination with vancomycin are effective at reducing *S*. *aureus* kidney burden at lower doses than rifampicin in combination with vancomycin (Example 3A). For example, rifamycin analog (II) reduced *S*. *aureus* kidney burden to below the limit of detection even at the lowest tested dose of 0.01 mg/kg.

### Example 3C: Median S. aureus NRS384 kidney burden in mice treated with rifampicin or rifamycin analog (II) with or without combination with vancomycin

In this experiment, mice infected with *S*. *aureus* MSRA strain NRS384 were treated with 25 mg/kg rifampicin (control) or 0.002 - 0.25 mg/kg rifamycin analog (II) alone or in combination with vancomycin, with vancomycin alone as a control.

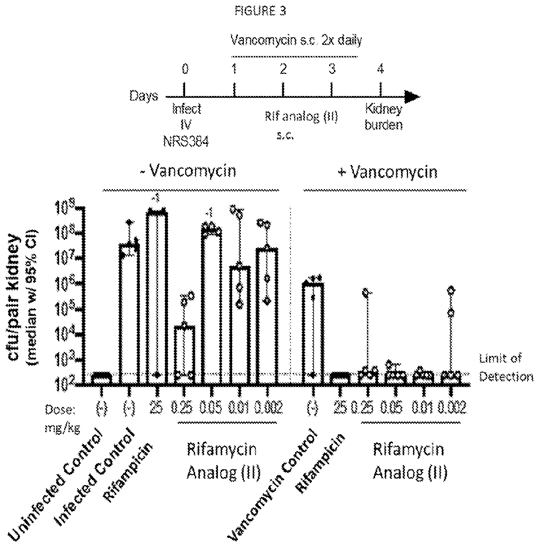

As shown in Figure 3 and Table 3, intravenous infection with *S*. *aureus* MRSA strain NRS384 results in high bacterial burden in the kidneys. Vancomycin treatment alone resulted in merely a ~2-3 log reduction in *S*. *aureus* kidney burden. Rifampicin reduced kidney burden at 25 mg/kg when combined with vancomycin, but was ineffective as a monotherapy. Rifamycin analog (II) reduced *S*. *aureus* kidney burden by ~3 logs at 0.25 mg/kg when administered as a monotherapy.

**Table 3: Median S. aureus NRS384 kidney burden in mice treated with rifampicin or rifamycin analog (II) with or without combination with vancomycin**

| **Treatment** | **Dose (mg/kg)** | **Vancomycin treatment** | **Median cfu/ kidney pair** | **Standard deviation** |
|---|---|---|---|---|
| Uninfected Control | | - | 2.50E+02 | 0.00E+00 |
| Infected Control | | - | 3.88E+07 | 1.10E+08 |
| Rifampicin | 25* | - | 7.07E+08 | 3.83E+08 |
| rifamycin analog (II) | 0.25 | - | 2.13E+04 | 1.50E+05 |
| | 0.05* | - | 1.52E+08 | 4.91E+07 |
| | 0.01 | - | 5.13E+06 | 4.13E+08 |
| | 0.002 | - | 2.75E+07 | 1.27E+08 |
| Vancomycin Control | 110 mg/kg twice daily | + | 1.13E+06 | 7.88E+05 |
| Rifampicin | 25 | + | 2.50E+02 | 0.00E+00 |
| rifamycin analog (II) | 0.25 | + | 3.75E+02 | 1.96E+05 |
| | 0.05 | + | 2.50E+02 | 1.68E+02 |
| | 0.01 | + | 2.50E+02 | 5.59E+01 |
| | 0.002 | + | 2.50E+02 | 2.34E+05 |
| *Mortality in 1/5 mice | | | Limit of detection = 250 | |

As shown in Table 3 and Figure 3, rifamycin analog (II) is highly effective at low doses, including 0.05 and 0.002 mg/kg, reducing the bacterial load to below the limit of detection when given in combination with vancomycin. Rifamycin analog (II) in combination with vancomycin is more effective than either monotherapy, i.e., than rifamycin analog (II) alone or vancomycin alone, in reducing the bacterial burden.

As shown in Table 3 and Figure 3, the efficacy of rifamycin analog (II) is enhanced by its use in combination with vancomycin. The efficacy of vancomycin is enhanced by its use in combination with rifamycin analog (II).

### Example 3D: Median S. aureus N315 kidney burden in mice treated with rifampicin or rifamycin analogs (I) or (II) in combination with vancomycin

In this experiment, mice infected with *S*. *aureus* MSRA strain N315 were treated with 25 mg/kg rifampicin (control), or 0.01 - 0.75 mg/kg rifamycin analog (I), or 0.002 - 0.25 mg/kg rifamycin analog (II) in combination with vancomycin, with vancomycin alone as a control.

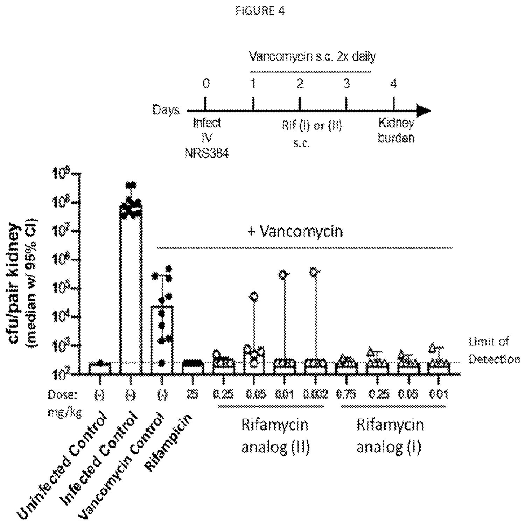

As shown in Figure 4 and Table 4, intravenous infection with *S*. *aureus* MRSA strain N315 results in high bacterial burden in the kidneys. Vancomycin treatment alone resulted in a ~4 log reduction in S. aureus kidney burden. Combination treatment of 25 mg/kg rifampicin or 0.01 - 0.75 mg/kg rifamycin analog (I) or 0.002 - 0.25 mg/kg rifamycin analog (II) with vancomycin further reduced kidney burden to at or near the limit of detection for the experiment.

**Table 4: Median S. aureus N315 kidney burden in mice treated with rifampicin or rifamycin analogs (I) or (II) in combination with vancomycin**

| **Treatment** | **Dose (mg/kg)** | **Vancomycin treatment** | **Median cfu/ kidney pair** | **Standard deviation** |
|---|---|---|---|---|
| Uninfected Control | | - | 250 | not calculated |
| Infected Control | | - | 7.50E+07 | 1.15E+08 |
| Vancomycin Control | 110 mg/kg twice daily | + | 3.88E+04 | 1.71E+05 |
| Rifampicin | 25 | + | 2.50E+02 | 0.00E+00 |
| Rifamycin analog (II) | 0.25 | + | 2.50E+02 | 1.12E+02 |
| | 0.05 | + | 6.25E+02 | 2.27E+04 |
| | 0.01 | + | 2.50E+02 | 1.34E+05 |
| | 0.002 | + | 2.50E+02 | 1.68E+05 |
| Rifamycin analog (I) | 0.75 | + | 2.50E+02 | 5.59E+01 |
| | 0.25 | + | 2.50E+02 | 1.68E+02 |
| | 0.05 | + | 2.50E+02 | 1.12E+02 |
| | 0.01 | + | 2.50E+02 | 2.80E+02 |
| No mortality in study | | | Limit of detection = 250 | |

Rifamycin analog (I) was efficacious in combination with vancomycin to reduce the bacterial load to below the limit of detection at a dose of 0.01 mg/kg. Rifamycin analog (II) was efficacious in combination with vancomycin to reduce the bacterial load to below the limit of detection at a dose of 0.002 mg/kg.

## Claims

1. Compound (a) or a pharmaceutically acceptable salt thereof, wherein compound (a) is selected from 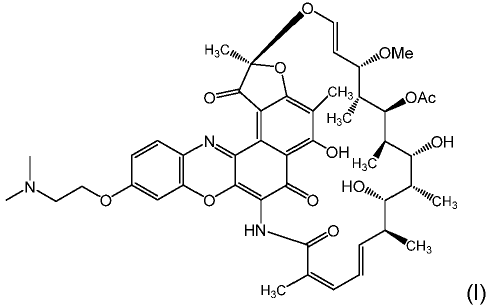 and 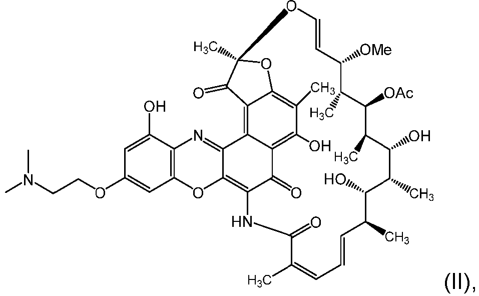 in combination with vancomycin or an ester, amide, N-oxide, a pharmaceutically acceptable salt of an ester or amide, or a pharmaceutically acceptable salt thereof, for use in a method of preventing or inhibiting growth of a bacterium.

2. The combination for use of claim 1, wherein the bacterium is a Gram-positive bacterium; and/or
wherein the bacterium is a penicillin-resistant bacterium, or a methicillin-resistant bacterium; and/or
wherein the bacterium is Staphylococcus aureus; preferably
wherein the bacterium is selected from methicillin-resistant Staphylococcus aureus (MRSA) and methicillin-susceptible Staphylococcus aureus (MSSA); more preferably wherein the bacterium is methicillin-resistant Staphylococcus aureus (MRSA).

3. Compound (a) or a pharmaceutically acceptable salt thereof, wherein compound (a) is selected from 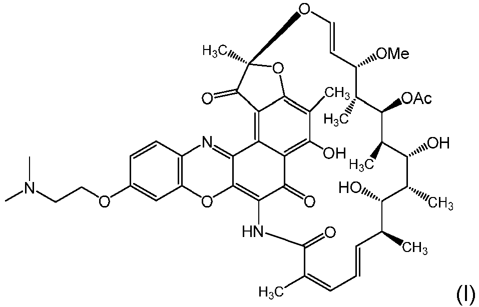 and 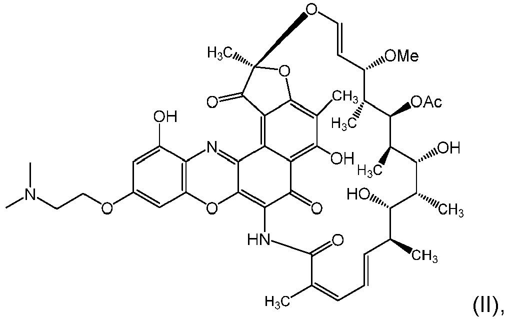 in combination with vancomycin or an ester, amide, N-oxide, a pharmaceutically acceptable salt of an ester or amide, or a pharmaceutically acceptable salt thereof, for use in a method of treating a bacterial infection in a subject.

4. The combination for use of any one of claims 1-3, wherein the compound (a) is compound (I) or a pharmaceutically acceptable salt thereof; or
wherein the compound (a) is compound (II) or a pharmaceutically acceptable salt thereof.

5. The combination for use of any one of claims 3-4, wherein the bacterial infection is caused by a Gram-positive bacterium; and/or
wherein the bacterial infection is caused by a penicillin-resistant bacterium or a methicillin-resistant bacterium; and/or
wherein the bacterial infection is caused by Staphylococcus aureus; preferably
wherein the bacterial infection is caused by a bacterium selected from methicillin-resistant Staphylococcus aureus (MRSA) and methicillin-susceptible Staphylococcus aureus (MSSA); more preferably
wherein the bacterial infection is caused by methicillin-resistant Staphylococcus aureus (MRSA); and/or
wherein the bacterial infection is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

6. Compound (a) or a pharmaceutically acceptable salt thereof, wherein compound (a) is selected from 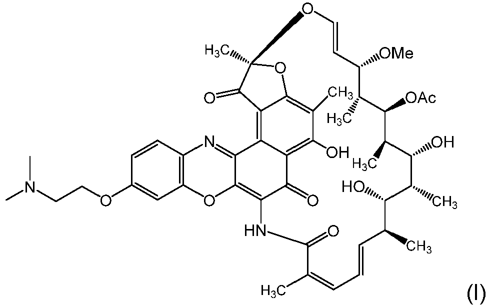 and 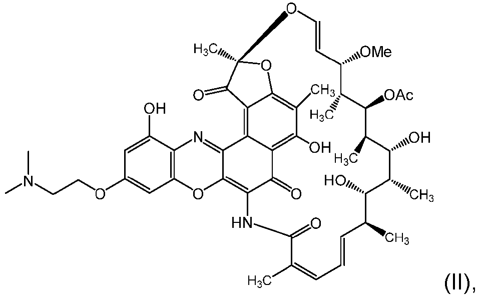
in combination with vancomycin or an ester, amide, N-oxide, or a pharmaceutically acceptable salt of an ester or amide, or a pharmaceutically acceptable salt thereof, for use in a method of preventing or treating a disease in a subject,
wherein the disease is selected from cellulitis, bacteremia, dermonecrosis, eyelid infection, eye infection, neonatal conjunctivitis, osteomyelitis, impetigo, boils, scalded skin syndrome, food poisoning, pneumonia, surgical infection, urinary tract infection, burn infection, meningitis, endocarditis, septicemia, toxic shock syndrome, septic arthritis, mastitis, infection associated with a prosthetic joint, infection associated with a catheter, and infection associated with an implant.

7. Compound (a) or a pharmaceutically acceptable salt thereof, wherein compound (a) is selected from 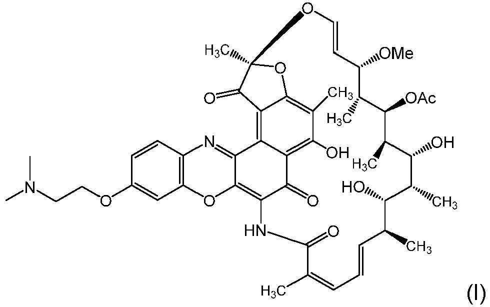 and 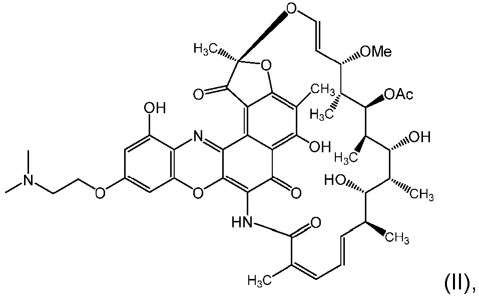
for use in a method of enhancing efficacy of vancomycin or an ester, amide, N-oxide, a pharmaceutically acceptable salt of an ester or amide, or a pharmaceutically acceptable salt thereof,
wherein compound (a) or a pharmaceutically acceptable salt thereof is administered to a subject in combination with vancomycin or an ester, amide, N-oxide, a pharmaceutically acceptable salt of an ester or amide, or a pharmaceutically acceptable salt thereof.

8. Vancomycin or an ester, amide, N-oxide, a pharmaceutically acceptable salt of an ester or amide, or a pharmaceutically acceptable salt thereof, for use in a method of enhancing efficacy of compound (a), wherein compound (a) is selected from 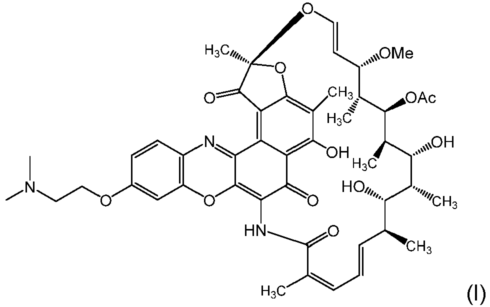 and 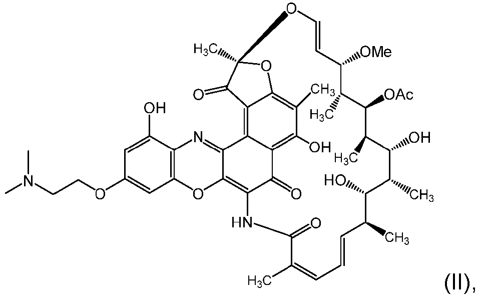
or a pharmaceutically acceptable salt thereof,
wherein the vancomycin or an ester, amide, N-oxide, a pharmaceutically acceptable salt of an ester or amide, or a pharmaceutically acceptable salt thereof is administered to a subject in combination with compound (a) or a pharmaceutically acceptable salt thereof.

9. The combination for use of any one of claims 1-6, the compound (a) or a pharmaceutically acceptable salt thereof for use of claim 7, or the vancomycin or an ester, amide, N-oxide, a pharmaceutically acceptable salt of an ester or amide, or a pharmaceutically acceptable salt thereof for use of claim 8, wherein the compound (I) or (II), or a pharmaceutically acceptable salt thereof, is administered orally, subcutaneously, or intravenously; and/or
wherein the vancomycin or an ester, amide, N-oxide, a pharmaceutically acceptable salt of an ester or amide, or a pharmaceutically acceptable salt thereof, is administered orally, subcutaneously, or intravenously.

10. The combination for use of any one of claims 1-6 or 9, the compound (a) or a pharmaceutically acceptable salt thereof for use of claim 7 or 9, or the vancomycin or an ester, amide, N-oxide, a pharmaceutically acceptable salt of an ester or amide, or a pharmaceutically acceptable salt thereof for use of claim 8 or 9, wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered in a single dosage form; or wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered in separate dosage forms and/or
wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on the same day; and/or
wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered simultaneously; and/or
wherein the vancomycin or an ester, amide, N-oxide, a pharmaceutically acceptable salt of an ester or amide, or a pharmaceutically acceptable salt thereof, is administered twice daily; or
wherein the compound (I) or (II), or a pharmaceutically acceptable salt thereof, is administered once daily.

11. The combination for use of any one of claims 1-6 or 9, the compound (a) or a pharmaceutically acceptable salt thereof for use of claim 7 or 9, or the vancomycin or an ester, amide, N-oxide, a pharmaceutically acceptable salt of an ester or amide, or a pharmaceutically acceptable salt thereof for use of claim 8 or 9, wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are not administered on the same day; preferably
wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on consecutive days; more preferably
wherein the compound (I) or (II), or a pharmaceutically acceptable salt thereof, and the vancomycin or an ester, amide, N-oxide, a pharmaceutically acceptable salt of an ester or amide, or a pharmaceutically acceptable salt thereof, are administered on separate, non-consecutive days; and/or
wherein the vancomycin or an ester, amide, N-oxide, a pharmaceutically acceptable salt of an ester or amide, or a pharmaceutically acceptable salt thereof, is administered twice daily; or
wherein the compound (I) or (II), or a pharmaceutically acceptable salt thereof, is administered once daily.

12. A pharmaceutical composition comprising a) a compound selected from 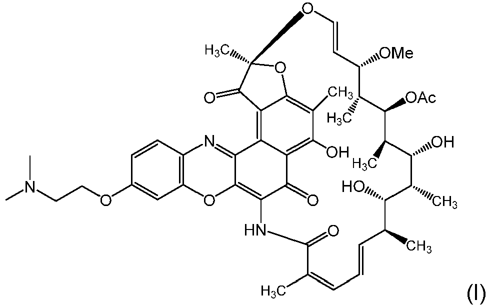 and 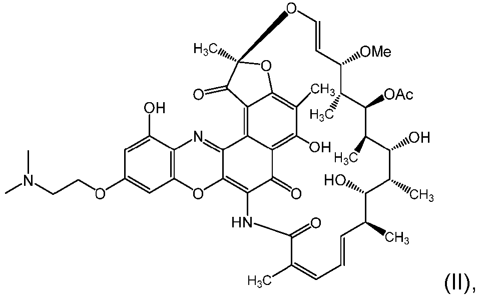
or a pharmaceutically acceptable salt thereof,
b) vancomycin or an ester, amide, N-oxide, a pharmaceutically acceptable salt of an ester or amide, or a pharmaceutically acceptable salt thereof, and
c) a pharmaceutically acceptable carrier.

13. A pharmaceutical dosage form comprising a) a compound selected from 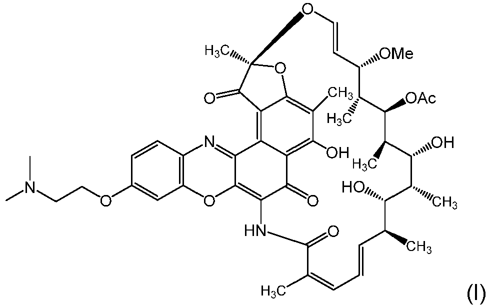 and 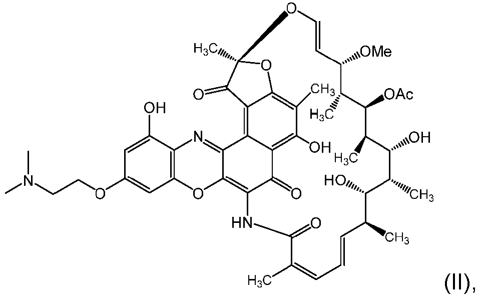
or a pharmaceutically acceptable salt thereof,
b) vancomycin or an ester, amide, N-oxide, a pharmaceutically acceptable salt of an ester or amide, or a pharmaceutically acceptable salt thereof, and
c) a pharmaceutically acceptable vehicle.

14. The pharmaceutical dosage form of claim 13, wherein the dosage form is an oral dosage form or an injectable dosage form.

15. A kit comprising a) a first container comprising a compound selected from 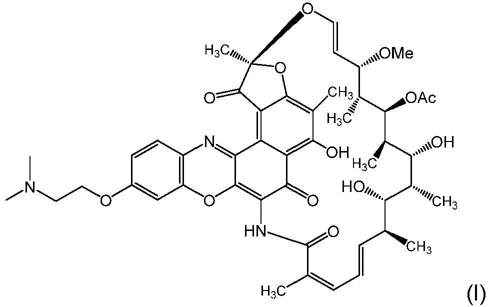 and 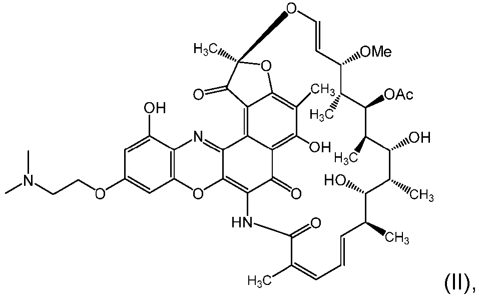
or a pharmaceutically acceptable salt thereof,
b) a second container comprising vancomycin or an ester, amide, N-oxide, a pharmaceutically acceptable salt of an ester or amide, or a pharmaceutically acceptable salt thereof, and
c) instructions for administration of the compound (I) or (II) and the vancomycin or an ester, amide, N-oxide, a pharmaceutically acceptable salt of an ester or amide, or a pharmaceutically acceptable salt thereof.

16. The kit of claim 15, wherein the first container comprises compound (I) or a pharmaceutically acceptable salt thereof; or
wherein the first container comprises compound (II) or a pharmaceutically acceptable salt thereof.

## Patentansprüche

1. Verbindung (a) oder pharmazeutisch unbedenkliches Salz davon, wobei die Verbindung (a) aus 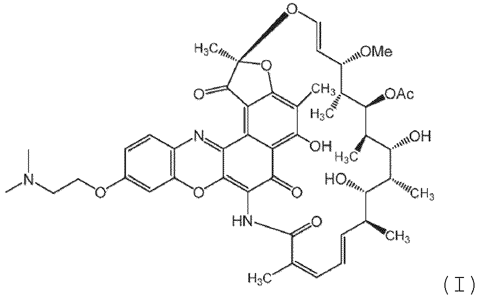 und 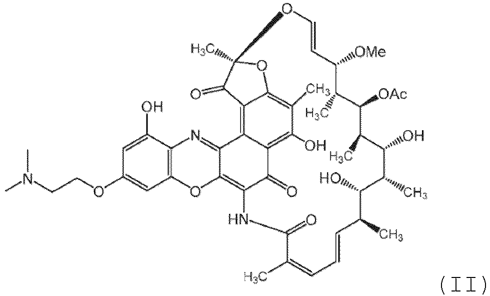 ausgewählt ist, in Kombination mit Vancomycin oder einem Ester, Amid, N-Oxid, einem pharmazeutisch unbedenklichen Salz eines Esters oder Amids, oder einem pharmazeutisch unbedenklichen Salz davon, zur Verwendung in einem Verfahren zur Verhütung oder Hemmung des Wachstums eines Bakteriums.

2. Kombination zur Verwendung nach Anspruch 1, wobei das Bakterium ein grampositives Bakterium ist; und/oder wobei das Bakterium ein Penicillin-resistentes Bakterium oder ein Methicillin-resistentes Bakterium ist; und/oder wobei es sich bei dem Bakterium um Staphylococcus aureus handelt; wobei das Bakterium vorzugsweise aus Methicillin-resistentem Staphylococcus aureus (MRSA) und Methicillin-empfindlihem Staphylococcus aureus (MSSA) ausgewählt ist; wobei es sich bei dem Bakterium insbesondere um Methicillin-resistenten Staphylococcus aureus (MRSA) handelt.

3. Verbindung (a) oder pharmazeutisch unbedenkliches Salz davon, wobei die Verbindung (a) aus 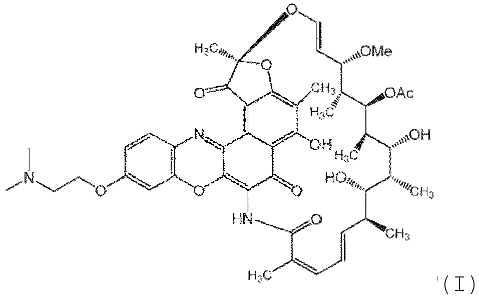 und 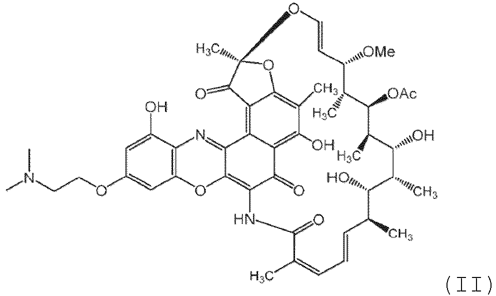 ausgewählt ist,
in Kombination mit Vancomycin oder einem Ester, Amid, N-Oxid, einem pharmazeutisch unbedenklichen Salz eines Esters oder Amids, oder einem pharmazeutisch unbedenklichen Salz davon, zur Verwendung in einem Verfahren zur Behandlung einer bakteriellen Infektion bei einem Zielorganismus.

4. Kombination zur Verwendung in einem beliebigen der Ansprüche 1 bis 3, wobei es sich bei der Verbindung (a) um eine Verbindung (I) oder ein pharmazeutisch unbedenkliches Salz einer solchen handelt; oder
wobei es sich bei der Verbindung (a) um eine Verbindung (II) oder ein pharmazeutisch unbedenkliches Salz einer solchen handelt.

5. Kombination zur Verwendung nach einem beliebigen der Ansprüche 3 bis 4, wobei die bakterielle Infektion durch ein grampositives Bakterium verursacht wird; und/oder wobei die bakterielle Infektion durch ein Penicillin-resistentes Bakterium oder ein Methicillin-resistentes Bakterium verursacht wird; und/oder
wobei die bakterielle Infektion durch Staphylococcus aureus verursacht wird; wobei die bakterielle Infektion vorzugsweise
durch ein Bakterium verursacht wird, das aus Methicillin-resistentem Staphylococcus aureus (MRSA) und Methicillin-empfindlichem Staphylococcus aureus (MSSA) ausgewählt ist; wobei die bakterielle Infektion insbesondere
durch Methicillin-resistenten Staphylococcus aureus (MRSA) verursacht wird; und/oder
wobei die bakterielle Infektion aus Zellulitis, Bakteriämie, Hautnekrose, Augenlidinfektionen, Augeninfektionen, neonataler Bindehautentzündung, Osteomyelitis, Impetigo contagiosa, Furunkeln, dem Scalded-Skin-Syndrome, Lebensmittelvergiftung, Lungenentzündung, Infektionen bei chirurgischen Eingriffen, Infektionen der Harnwege, verbrennungsbedingten Infektionen, Hirnhautentzündung, Endokarditis, Sepsis, dem toxischen Schocksyndrom, septischer Arthritis, Mastitis, Infektionen in Verbindung mit einer Gelenkprothese, Infektionen in Verbindung mit einem Katheter und Infektionen in Verbindung mit einem Implantat ausgewählt ist.

6. Verbindung (a) oder pharmazeutisch unbedenkliches Salz davon, wobei die Verbindung (a) aus 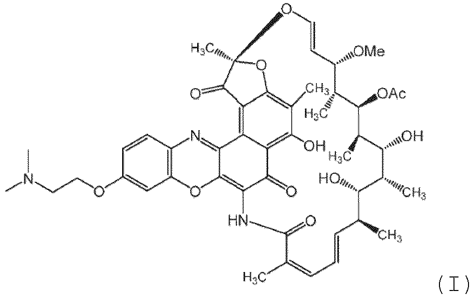 und 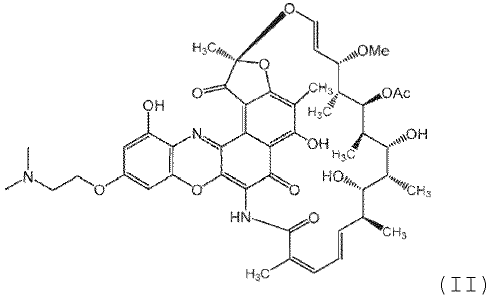 ausgewählt ist,
in Kombination mit Vancomycin oder einem Ester, Amid, **N-**Oxid, einem pharmazeutisch unbedenklichen Salz eines Esters oder Amids, oder einem pharmazeutisch unbedenklichen Salz davon, zur Verwendung in einem Verfahren zur Verhütung oder Behandlung einer Krankheit bei einem Zielorganismus,
wobei die Krankheit aus Zellulitis, Bakteriämie, Hautnekrose, Augenlidinfektionen, Augeninfektionen, neonataler Bindehautentzündung, Osteomyelitis, Impetigo contagiosa, Furunkeln, dem Scalded-Skin-Syndrome, Lebensmittelvergiftung, Lungenentzündung, Infektionen bei chirurgischen Eingriffen, Infektionen der Harnwege, verbrennungsbedingten Infektionen, Hirnhautentzündung, Endokarditis, Sepsis, dem toxischen Schocksyndrom, septischer Arthritis, Mastitis, Infektionen in Verbindung mit einer Gelenkprothese, Infektionen in Verbindung mit einem Katheter und Infektionen in Verbindung mit einem Implantat ausgewählt ist.

7. Verbindung (a) oder pharmazeutisch unbedenkliches Salz davon, wobei die Verbindung (a) aus 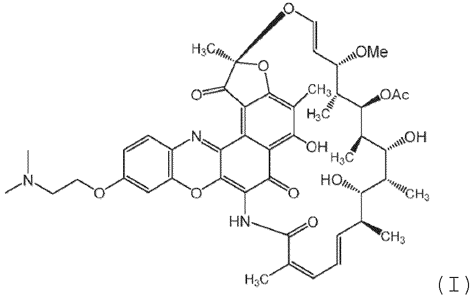 und 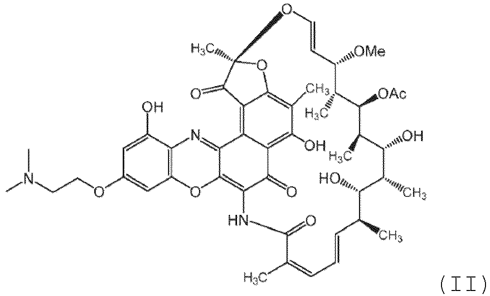 ausgewählt ist,
zur Verwendung in einem Verfahren zur Verstärkung der Wirksamkeit von Vancomycin oder eines Esters, Amids, **N-**Oxids, eines pharmazeutisch unbedenkliches Salzes eines Esters oder Amids, oder eines pharmazeutisch unbedenklichen Salzes davon,
wobei die Verbindung (a) oder ein pharmazeutisch unbedenkliches Salz derselben einem Zielorganismus in Kombination mit Vancomycin oder einem Ester, Amid, N-Oxid, einem pharmazeutisch unbedenklichen Salz eines Esters oder Amids, oder einem pharmazeutisch unbedenklichen Salz davon verabreicht wird.

8. Vancomycin oder ein Ester, Amid, N-Oxid, ein pharmazeutisch unbedenkliches Salz eines Esters oder Amids, oder ein pharmazeutisch unbedenkliches Salz davon, zur Verwendung in einem Verfahren zur Verstärkung der Wirksamkeit einer Verbindung (a), wobei die Verbindung (a) aus 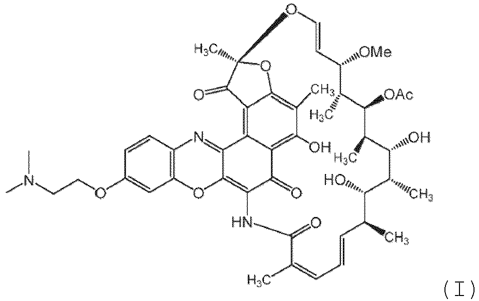 und 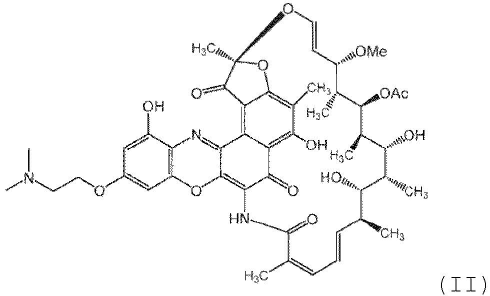
oder einem pharmazeutisch unbedenklichen Salz davon ausgewählt ist,
wobei das Vancomycin oder ein Ester, Amid, N-Oxid, ein pharmazeutisch unbedenkliches Salz eines Esters oder Amids, oder ein pharmazeutisch unbedenkliches Salz davon einem Zielorganismus in Kombination mit Verbindung (a) oder einem pharmazeutisch unbedenklichen Salz davon verabreicht wird.

9. Kombination zur Verwendung nach einem beliebigen der Ansprüche 1 bis 6, Verbindung (a) oder ein pharmazeutisch unbedenkliches Salze davon zur Verwendung nach Anspruch 7, oder Vancomycin oder ein Ester, Amid, N-Oxid, ein pharmazeutisch unbedenkliches Salz eines Esters oder Amids, oder ein pharmazeutisch unbedenkliches Salz davon zur Verwendung nach Anspruch 8, wobei die Verbindung (I) oder (II), oder ein pharmazeutisch unbedenkliches Salz davon, oral, subkutan oder intravenös verabreicht wird; und/oder
wobei das Vancomycin oder ein Ester, Amid, N-Oxid, ein pharmazeutisch unbedenkliches Salz eines Esters oder Amids, oder ein pharmazeutisch unbedenkliches Salz davon oral, subkutan oder intravenös verabreicht wird.

10. Kombination zur Verwendung nach einem beliebigen der Ansprüche 1 bis 6 oder 9, Verbindung (a) oder ein pharmazeutisch unbedenkliches Salz davon zur Verwendung nach Anspruch 7 oder 9, oder Vancomycin oder ein Ester, Amid, N-Oxid, ein pharmazeutisch unbedenkliches Salz eines Esters oder Amids, oder ein pharmazeutisch unbedenkliches Salz davon zur Verwendung nach Anspruch 8 oder 9, wobei die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, in einer Einheitsdosisform verabreicht werden; oder wobei die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, in separaten Darreichungsformen verabreicht werden, und/oder
wobei die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, am selben Tag verabreicht werden; und/oder
wobei die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, gleichzeitig verabreicht werden; und/oder
wobei das Vancomycin oder ein Ester, Amid, N-Oxid, ein pharmazeutisch unbedenkliches Salz eines Esters oder Amids, oder ein pharmazeutisch unbedenkliches Salz davon zweimal täglich verabreicht wird; oder
wobei die Verbindung (I) oder (II), oder ein pharmazeutisch unbedenklichen Salz davon, einmal täglich verabreicht wird.

11. Kombination zur Verwendung nach einem beliebigen der Ansprüche 1 bis 6 oder 9, Verbindung (a) oder ein pharmazeutisch unbedenkliches Salz davon zur Verwendung nach Anspruch 7 oder 9, oder Vancomycin oder ein Ester, Amid, N-Oxid, ein pharmazeutisch unbedenkliches Salz eines Esters oder Amids, oder ein pharmazeutisch unbedenkliches Salz davon zur Verwendung nach Anspruch 8 oder 9, wobei die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, nicht am selben Tag verabreicht werden; wobei vorzugsweise
die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, an aufeinander folgenden Tagen werden; wobei insbesondere
die Verbindung (I) oder (II), oder ein pharmazeutisch unbedenkliches Salz davon, und das Vancomycin oder ein Ester, Amid, N-Oxid, ein pharmazeutisch unbedenkliches Salz eines Esters oder Amids, oder ein pharmazeutisch unbedenkliches Salz davon, an gesonderten, nicht aufeinander folgenden Tagen verabreicht werden; und/oder wobei das Vancomycin oder ein Ester, Amid, N-Oxid, ein pharmazeutisch unbedenkliches Salz eines Esters oder Amids, oder ein pharmazeutisch unbedenkliches Salz davon zweimal täglich verabreicht wird; oder
wobei die Verbindung (I) oder (II), oder ein pharmazeutisch unbedenklichen Salz davon, einmal täglich verabreicht wird.

12. Pharmazeutische Zusammensetzung, die a) eine Verbindung, welche aus 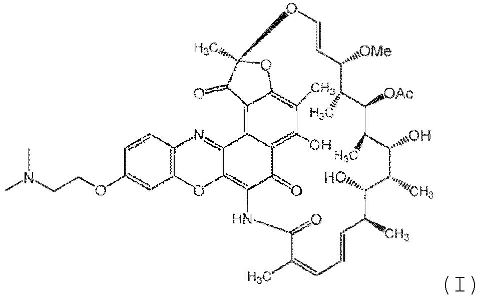 und 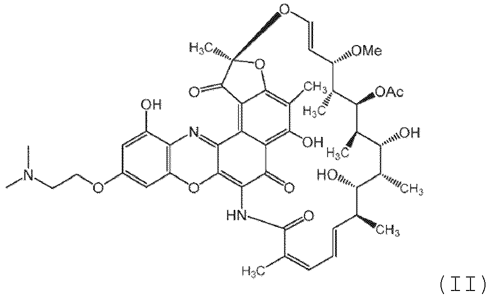
oder einem pharmazeutisch unbedenklichen Salz davon ausgewählt ist,
b) Vancomycin oder einen Ester, ein Amid, ein N-Oxid, ein pharmazeutisch unbedenkliches Salz eines Esters oder Amids, oder ein pharmazeutisch unbedenkliches Salz davon, und
c) einen pharmazeutisch unbedenklichen Trägerstoff umfasst.

13. Pharmazeutische Darreichungsform, die a) eine Verbindung, welche aus 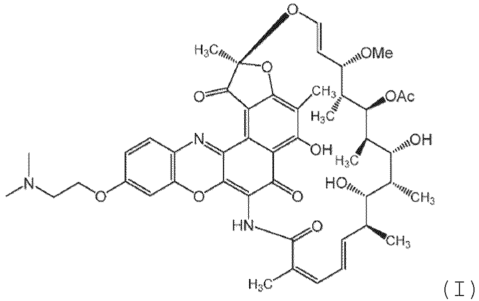 und 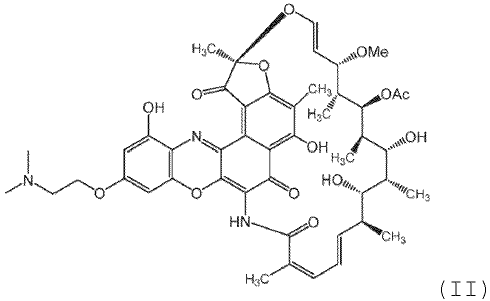
oder einem pharmazeutisch unbedenklichen Salz davon ausgewählt ist,
b) Vancomycin oder einen Ester, ein Amid, ein N-Oxid, ein pharmazeutisch unbedenkliches Salz eines Esters oder Amids, oder ein pharmazeutisch unbedenkliches Salz davon, und
c) einen pharmazeutisch unbedenklichen Träger umfasst.

14. Pharmazeutische Darreichungsform nach Anspruch 13, wobei es sich bei der Darreichungsform um eine orale Darreichungsform oder eine Darreichungsform zur Injektion handelt.

15. Kit, das a) einen ersten Behälter, welcher eine Verbindung umfasst, die aus 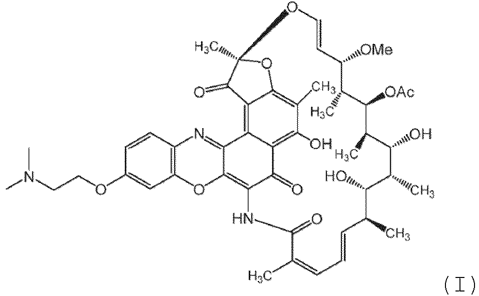 und 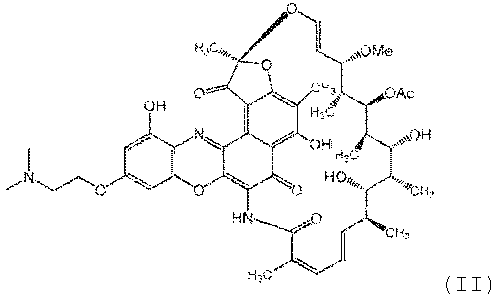
oder einem pharmazeutisch unbedenklichen Salz davon ausgewählt ist,
b) einen zweiten Behälter, welcher Vancomycin oder einen Ester, ein Amid, ein N-Oxid, ein pharmazeutisch unbedenkliches Salz eines Esters oder Amids, oder ein pharmazeutisch unbedenkliches Salz davon umfasst, und
c) Anweisungen zur Verabreichung der Verbindung (I) oder (II) und des Vancomycins oder eines Esters, Amids, N-Oxids, eines pharmazeutisch unbedenklichen Salzes eines Esters oder Amids, oder eines pharmazeutisch unbedenklichen Salzes davon umfasst.

16. Kit nach Anspruch 15, wobei der erste Behälter Verbindung (I) oder ein pharmazeutisch unbedenkliches Salz davon umfasst; oder
wobei der erste Behälter Verbindung (II) oder ein pharmazeutisch unbedenkliches Salz davon umfasst.

## Revendications

1. Composé (a) ou sel pharmaceutiquement acceptable de celui-ci, dans lequel le composé (a) est choisi parmi 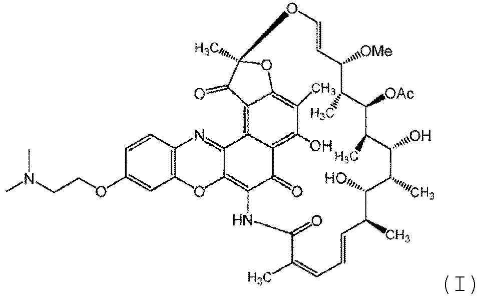 et 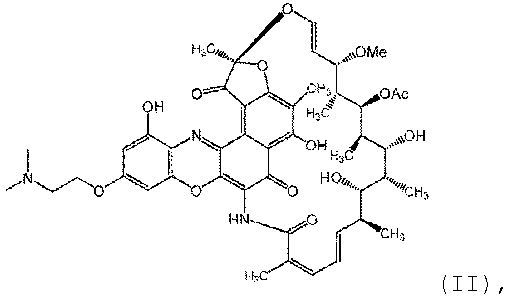 en combinaison avec de la vancomycine ou un ester, amide, N-oxyde, un sel pharmaceutiquement acceptable d'un ester ou amide, ou un sel pharmaceutiquement acceptable correspondant, pour une utilisation dans un procédé de prévention ou d'inhibition de la croissance d'une bactérie.

2. Combinaison pour une utilisation selon la revendication 1, dans laquelle la bactérie est une bactérie Gram-positive ; et/ou
dans laquelle la bactérie est une bactérie résistante à la pénicilline ou une bactérie résistante à la méthicilline ; et/ou
dans laquelle la bactérie est Staphylococcus aureus ; de préférence
dans laquelle la bactérie est choisie parmi Staphylococcus aureus résistant à la méthicilline (MRSA) et Staphylococcus aureus sensible à la méthicilline (MSSA) ; plus préférablement, dans laquelle la bactérie est Staphylococcus aureus résistant à la méthicilline (MRSA).

3. Composé (a) ou sel pharmaceutiquement acceptable de celui-ci, dans lequel le composé (a) est choisi parmi 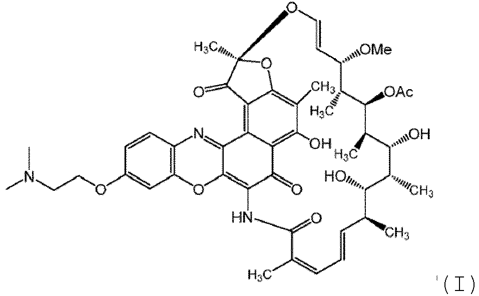 et 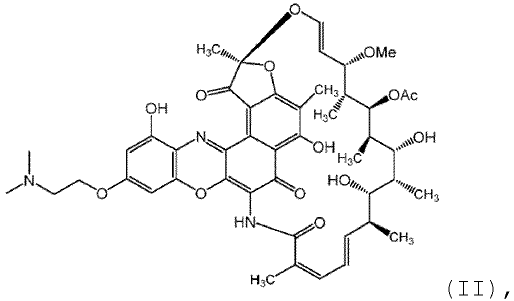 en combinaison avec de la vancomycine ou un ester, amide, N-oxyde, un sel pharmaceutiquement acceptable d'un ester ou amide, ou un sel pharmaceutiquement acceptable correspondant, pour une utilisation dans un procédé de traitement d'une infection bactérienne chez un sujet.

4. Combinaison pour une utilisation selon l'une quelconque des revendications 1 à 3, dans laquelle le composé (a) est le composé (I) ou un sel pharmaceutiquement acceptable de celui-ci ; ou
dans laquelle le composé (a) est le composé (II) ou un sel pharmaceutiquement acceptable de celui-ci.

5. Combinaison pour une utilisation selon l'une quelconque des revendications 3 à 4, dans laquelle l'infection bactérienne est provoquée par une bactérie Gram-positive ; et/ou
dans laquelle l'infection bactérienne est provoquée par une bactérie résistante à la pénicilline ou une bactérie résistante à la méthicilline ; et/ou
dans laquelle l'infection bactérienne est provoquée par Staphylococcus aureus ; de préférence
dans laquelle l'infection bactérienne est provoquée par une bactérie choisie parmi Staphylococcus aureus résistant à la méthicilline (MRSA) et Staphylococcus aureus sensible à la méthicilline (MSSA) ; plus préférablement
dans laquelle l'infection bactérienne est provoquée par Staphylococcus aureus résistant à la méthicilline (MRSA) ; et/ou
dans laquelle l'infection bactérienne est choisie parmi la cellulite, la bactériémie, la dermonécrose, l'infection des paupières, l'infection oculaire, la conjonctivite néonatale, l'ostéomyélite, l'impétigo, les furoncles, le syndrome de la peau ébouillantée, l'intoxication alimentaire, la pneumonie, l'infection chirurgicale, l'infection des voies urinaires, l'infection des brûlures, la méningite, l'endocardite, la septicémie, le syndrome du choc toxique, l'arthrite septique, la mastite, l'infection associée à une prothèse articulaire, l'infection associée à un cathéter et l'infection associée à un implant.

6. Composé (a) ou sel pharmaceutiquement acceptable de celui-ci, dans lequel le composé (a) est choisi parmi 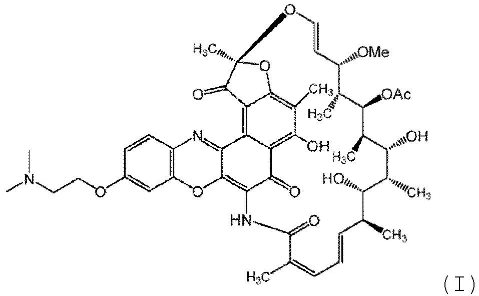 et 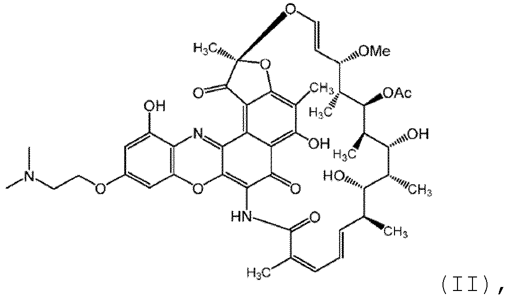 en combinaison avec de la vancomycine ou un ester, amide, N-oxyde, ou un sel pharmaceutiquement acceptable d'un ester ou amide, ou un sel pharmaceutiquement acceptable correspondant, pour une utilisation dans un procédé de prévention ou de traitement d'une maladie chez un sujet, dans lequel la maladie est choisie parmi la cellulite, la bactériémie, la dermonécrose, l'infection des paupières, l'infection oculaire, la conjonctivite néonatale, l'ostéomyélite, l'impétigo, les furoncles, le syndrome de la peau ébouillantée, l'intoxication alimentaire, la pneumonie, l'infection chirurgicale, l'infection des voies urinaires, l'infection des brûlures, la méningite, l'endocardite, la septicémie, le syndrome du choc toxique, l'arthrite septique, la mastite, l'infection associée à une prothèse articulaire, l'infection associée à un cathéter et l'infection associée à un implant.

7. Composé (a) ou sel pharmaceutiquement acceptable de celui-ci, dans lequel le composé (a) est choisi parmi 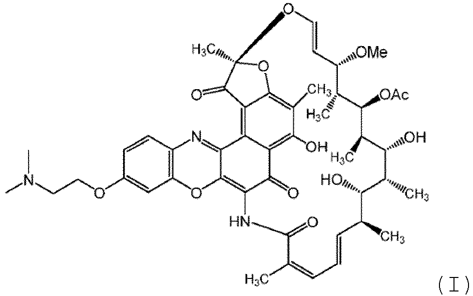 et 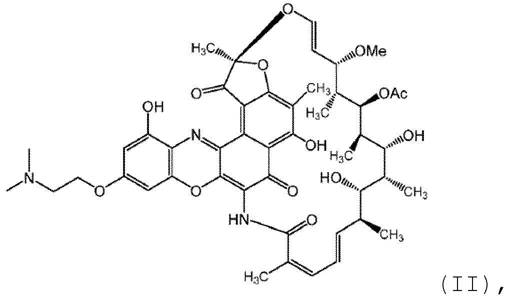
pour une utilisation dans un procédé d'amélioration de l'efficacité de la vancomycine ou d'un ester, amide, N-oxyde, d'un sel pharmaceutiquement acceptable d'un ester ou amide, ou d'un sel pharmaceutiquement acceptable correspondant,
dans lequel le composé (a) ou un sel pharmaceutiquement acceptable de celui-ci est administré à un sujet en combinaison avec de la vancomycine ou un ester, amide, N-oxyde, un sel pharmaceutiquement acceptable d'un ester ou amide, ou un sel pharmaceutiquement acceptable correspondant.

8. Vancomycine ou ester, amide, N-oxyde, sel pharmaceutiquement acceptable d'un ester ou amide, ou sel pharmaceutiquement acceptable correspondant, pour une utilisation dans un procédé d'amélioration de l'efficacité du composé (a), dans laquelle/lequel le composé (a) est choisi parmi 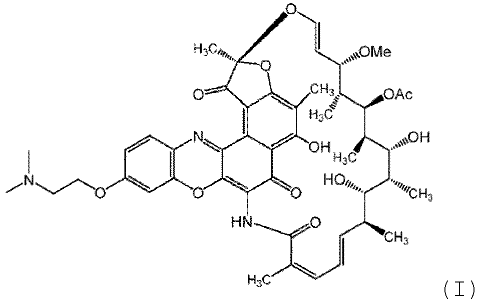 et 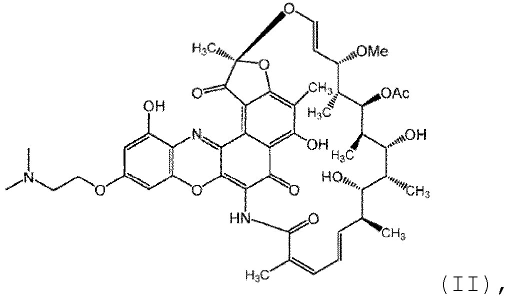 ou un sel pharmaceutiquement acceptable de celui-ci, dans laquelle/lequel la vancomycine ou un ester, amide, N-oxyde, un sel pharmaceutiquement acceptable d'un ester ou amide, ou un sel pharmaceutiquement acceptable correspondant, est administré(e) à un sujet en combinaison avec le composé (a) ou un sel pharmaceutiquement acceptable de celui-ci.

9. Combinaison pour une utilisation selon l'une quelconque des revendications 1 à 6, composé (a) ou sel pharmaceutiquement acceptable de celui-ci pour une utilisation selon la revendication 7, ou vancomycine ou ester, amide, N-oxyde, sel pharmaceutiquement acceptable d'un ester ou amide, ou sel pharmaceutiquement acceptable correspondant pour une utilisation selon la revendication 8, dans laquelle/lequel le composé (I) ou (II), ou un sel pharmaceutiquement acceptable de celui-ci, est administré par voie orale, sous-cutanée ou intraveineuse ; et/ou
dans laquelle/lequel la vancomycine ou un ester, amide, N-oxyde, un sel pharmaceutiquement acceptable d'un ester ou amide, ou un sel pharmaceutiquement acceptable correspondant, est administré(e) par voie orale, sous-cutanée ou intraveineuse.

10. Combinaison pour une utilisation selon l'une quelconque des revendications 1-6 ou 9, composé (a) ou sel pharmaceutiquement acceptable de celui-ci pour une utilisation selon la revendication 7 ou 9, ou vancomycine ou ester, amide, N-oxyde, sel pharmaceutiquement acceptable d'un ester ou amide, ou sel pharmaceutiquement acceptable correspondant pour une utilisation selon la revendication 8 ou 9, dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés sous une forme posologique unique ; ou dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés sous des formes posologiques séparées et/ou dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés le même jour ; et/ou
dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés simultanément ; et/ou
dans laquelle/lequel la vancomycine ou un ester, amide, N-oxyde, un sel pharmaceutiquement acceptable d'un ester ou amide, ou un sel pharmaceutiquement acceptable correspondant, est administré(e) deux fois par jour ; ou dans laquelle/lequel le composé (I) ou (II), ou un sel pharmaceutiquement acceptable de celui-ci, est administré une fois par jour.

11. Combinaison pour une utilisation selon l'une quelconque des revendications 1-6 ou 9, composé (a) ou sel pharmaceutiquement acceptable de celui-ci pour une utilisation selon la revendication 7 ou 9, ou vancomycine ou ester, amide, N-oxyde, sel pharmaceutiquement acceptable d'un ester ou amide, ou sel pharmaceutiquement acceptable correspondant pour une utilisation selon la revendication 8 ou 9, dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, ne sont pas administrés le même jour ; de préférence
dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés pendant des jours consécutifs ; plus préférablement
dans laquelle/lequel le composé (I) ou (II), ou un sel pharmaceutiquement acceptable de celui-ci, et la vancomycine ou un ester, amide, N-oxyde, un sel pharmaceutiquement acceptable d'un ester ou amide, ou un sel pharmaceutiquement acceptable correspondant, sont administrés sur des jours séparés, non consécutifs ; et/ou
dans laquelle/lequel la vancomycine ou un ester, amide, N-oxyde, un sel pharmaceutiquement acceptable d'un ester ou amide, ou un sel pharmaceutiquement acceptable correspondant, est administré(e) deux fois par jour ; ou dans laquelle/lequel le composé (I) ou (II), ou un sel pharmaceutiquement acceptable de celui-ci, est administré une fois par jour.

12. Composition pharmaceutique comprenant a) un composé choisi parmi 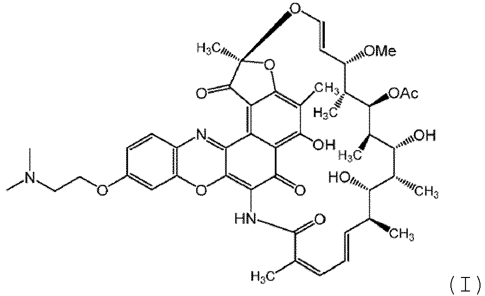 et 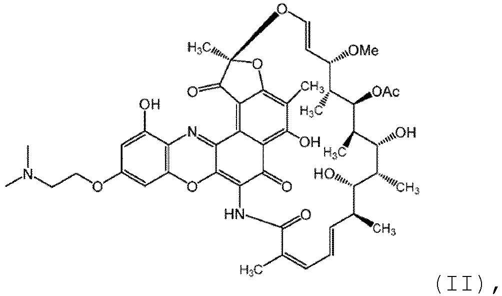
ou un sel pharmaceutiquement acceptable de celui-ci,
b) de la vancomycine ou un ester, amide, N-oxyde, un sel pharmaceutiquement acceptable d'un ester ou amide, ou un sel pharmaceutiquement acceptable correspondant, et
c) un support pharmaceutiquement acceptable.

13. Forme posologique pharmaceutique comprenant a) un composé choisi parmi 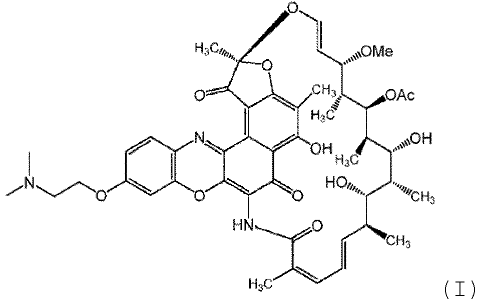 et 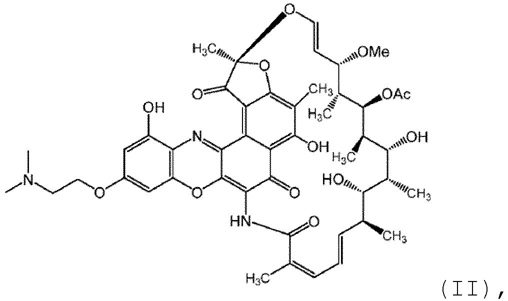
ou un sel pharmaceutiquement acceptable de celui-ci,
b) de la vancomycine ou un ester, amide, N-oxyde, un sel pharmaceutiquement acceptable d'un ester ou amide, ou un sel pharmaceutiquement acceptable correspondant, et
c) un véhicule pharmaceutiquement acceptable.

14. Forme posologique pharmaceutique selon la revendication 13, dans laquelle la forme posologique est une forme posologique orale ou une forme posologique injectable.

15. Kit comprenant a) un premier récipient comprenant un composé choisi parmi 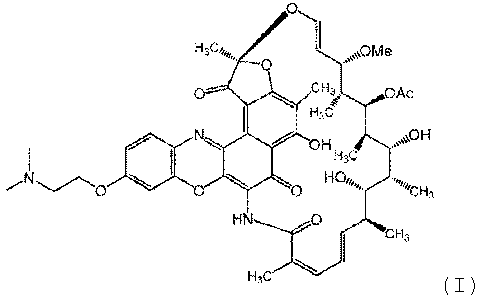 et 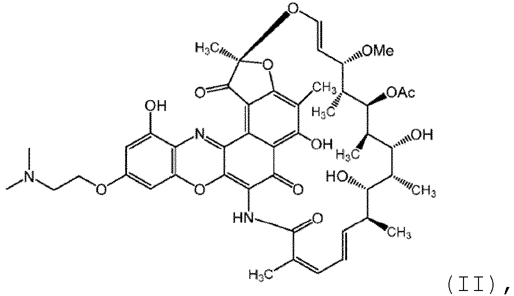
ou un sel pharmaceutiquement acceptable de celui-ci,
b) un second récipient comprenant de la vancomycine ou un ester, amide, N-oxyde, un sel pharmaceutiquement acceptable d'un ester ou amide, ou un sel pharmaceutiquement acceptable correspondant, et
c) des instructions pour l'administration du composé (I) ou (II) et de la vancomycine ou d'un ester, amide, N-oxyde, d'un sel pharmaceutiquement acceptable d'un ester ou amide, ou d'un sel pharmaceutiquement acceptable correspondant.

16. Kit selon la revendication 15, dans lequel le premier récipient comprend le composé (I) ou un sel pharmaceutiquement acceptable de celui-ci ; ou
dans lequel le premier récipient comprend le composé (II) ou un sel pharmaceutiquement acceptable de celui-ci.
